# FASCICULE DE BREVET EUROPEEN

(11) **EP 0 965 045 B1**
(45) Date de publication et mention de la délivrance du brevet: **09.01.2002**
(21) Numéro de dépôt: 98905473.9
(22) Date de dépôt: 30.01.1998
(51) Int. Cl.: G01N 33/553, G01N 33/548, G01N 33/543, G01N 33/76

(54) **PROCEDE POUR ISOLER, NOTAMMENT DETECTER OU QUANTIFIER UN ANALYTE DANS UN MILIEU**
VERFAHREN ZUR ISOLIERUNG, DETEKTION ODER QUANTIFIZIERUNG EINES ANALYTEN IN EINEM MEDIUM
METHOD FOR ISOLATING, IN PARTICULAR FOR DETECTING OR QUANTIFYING AN ANALYTE IN A MEDIUM

(30) Priorité: 30.01.1997 FR 9701313
(43) Date de publication de la demande: 22.12.1999
(73) Titulaire: BIO MERIEUX, 69280 Marcy l'Etoile (FR)
(72) Inventeur: PERRIN, Agnès, F-69007 Lyon (FR); THERETZ, Alain, F-69130 Ecully (FR); MANDRAND, Bernard, F-69100 Villeurbanne (FR)
(74) Mandataire: Guerre, Dominique
(86) Numéro de dépôt international: FR9800182
(87) Numéro de publication internationale: WO9834116

(56) Documents cités:
- WO-A-96/09409
- WO-A-97/07243
- DE-A- 4 036 288
- FR-A- 2 679 660
- US-A- 4 375 407
- US-A- 4 452 773

## Description

La présente invention concerne un procédé pour isoler au moins un analyte dans un milieu liquide, dans lequel ce dernier est distribué.

Par "isoler" ou "isolement", on entend de manière générique toute technique permettant de séparer ledit analyte, mais aussi d'enrichir ou concentrer en ledit analyte tout mélange liquide le contenant. Mais on entend aussi, éventuellement conjointement avec la définition précédente, toute technique permettant de déterminer l'analyte, au sens d'une détection et/ou quantification de celui-ci, à partir du milieu liquide le contenant.

Par "analyte", on entend toute entité, notamment entité biologique, à isoler. Au rang des analytes considérés ci-après par la présente invention, on citera les cellules, organelles, virus et bactéries, les anticorps, les fragments d'anticorps, les antigènes, les haptènes, les lectines, les sucres, les acides ribo-, déoxyribonucléiques, les protéines, notamment A ou G, les hormones, les récepteurs d'hormones, la biotine, l'avidine, la streptavidine, et d'une manière générale toutes molécules ou macromolécules, naturelles ou synthétiques, ou analogues, à déterminer, c'est-à-dire détecter et/ou quantifier.

Conformément au doçument FR-A-2 679 660, on connaît un procédé pour isoler au moins un analyte, à savoir soit un antigène libre ou lié à la surface d'une cellule, soit un anticorps dirigé contre un antigène cellulaire ou tissulaire, par exemple anticorps anti-érythrocytaire.

Selon de procédé de FR-A-2 679 660, on dispose d'au moins un réactif comprenant, d'une part un support libre, sous forme discrète, en l'occurrence des particules magnétiques de taille relativement importante, constituées par un polymère qui encapsule des grains de ferrite, et d'autre part exhibant un récepteur de l'analyte, à savoir soit un anticorps, soit un antigène ou substance révélatrice, par exemple un anticorps anti-immunoglobuline humaine.

On dispose aussi d'un autre support, de capture, constitué par la paroi d'un récipient ou cupule, dont la surface accessible comprend au moins une zone utile ou active, de surface limitée, exhibant au moins un autre récepteur d'une entité à capturer, à savoir dudit analyte ou dudit récepteur. Cet autre récepteur est soit un anticorps, soit un antigène, par exemple érythrocytaire.

Toujours selon ce procédé, on met en contact la surface accessible dudit autre support avec l'analyte, contenu dans un échantillon liquide, pour lier l'analyte à la surface accessible, et le rassembler sous la forme d'un dépôt sur ladite surface, immobilisé sur ledit autre support par l'intermédiaire dudit autre récepteur.

Puis, à des fins de révélation, on met au contact le réactif avec l'analyte immobilisé, pour obtenir avec ce dernier une association, également immobilisée sur ledit autre support et pouvant permettre l'isolement ou détermination de l'analyte.

Un procédé tel que défini précédemment est donc mis en oeuvre avec un réactif constitué par un support sous forme discrète, auquel est lié au moins un ligand formant un récepteur de l'analyte.

Préférentiellement, ces particules sont magnétiques. Elles peuvent. être rangées en deux catégories, à savoir les particules de diamètre relativement important, par exemple de l'ordre du ou de quelques microns, et celles de diamètre relativement faible, par exemple de l'ordre de quelques dizaines de nanomètres, et à l'état colloïdal.

Les particules magnétiques de diamètre relativement important lorsqu'elles sont placées dans un champ magnétique, se déplacent en direction de l'endroit où le champ est le plus élevé et à une vitesse suffisante pour être séparées de leur milieu par ce moyen.

A titre d'exemple, on peut citer les particules décrites dans le document EP-A-0 125 995. Elles sont obtenues par précipitation de sels ferreux et ferriques en milieu basique, suivie d'une réaction de silanisation dans le méthanol. Leur diamètre final est compris entre 0,1 et 1,5 µm et leur densité de 2,5 g/cm³. De même, les particules décrites dans les documents EP-A-0 106 873, EP-A-0 585 868 et US-C-5,356,713, sont obtenues par différents procédés de polymérisation, ou encore celles décrites dans le document US-C-4,297,337 utilisent une matrice de verre poreuse dans laquelle sont dispersés des pigments magnétiques. D'autres brevets décrivent aussi l'utilisation de particules de faibles tailles, mais volontairement agrégées afin d'augmenter la masse magnétique comme dans le document US-C-5,169,754. L'article de P.A. Risφen et al , Protein Expression and Purification, 6 (1995), 272-277 décrit aussi des gels magnétiques.

Placées dans un champ magnétique, toutes ces particules relativement grosses engendrent un mouvement en direction de l'endroit où le champ est le plus intense. Un simple aimant permanent ou des montages équivalent tels que décrits par exemple dans le document EP-A-0 317 286 ou US-C-565 665 peuvent être utilisés. Ces particules sont couramment employées pour la séparation de cellules ou de molécules, ainsi que dans des immuno-essais tels que décrit dans le document EP-A-0 528 708 mais ne sont pas utilisées en tant que système de détection et quantification.

Par ailleurs, leur diamètre et leur densité font qu'elles sédimentent très rapidement sous l'effet de la gravité, ce qui les rend difficiles à utiliser en raison des contraintes d'agitation ou de manque d'homogénéité des solutions dû à la création de gradient de concentration.

En résumé, les particules relativement grosses sont d'une mise en oeuvre délicate, et peu adaptées à la détermination d'un analyte.

Les particules magnétiques de diamètre relativement faible ne sont pratiquement pas attirées par un simple aimant permanent dans des délais raisonnables: Ces particules sont en particulier largement utilisées pour la séparation magnétique de cellules. Ces particules sont connues par l'homme de l'art sous l'appellation de particules "superparamagnétiques". Par particules "superparamagnétiques", on entend des particules dont le diamètre est trop faible pour être constituées de plusieurs domaines magnétiques. Elles se caractérisent par une forte susceptibilité magnétique et une magnétisation à saturation importante, mais une rémanence magnétique nulle ou très faible. Ces particules sont en particulier largement utilisées pour la séparation magnétique de cellule. Par exemple, celles décrites dans le document US-C-4,230,685 sont obtenues par émulsion d'un mélange d'albumine, de protéine A et de particules de Fe₃O₄ de diamètre 15-20 nm et peuvent immobiliser des anticorps par l'intermédiaire de la protéine A. Le document US-C-4,452,773 décrit un autre type de particules, obtenu par précipitation de sels ferreux et ferrique en milieu basique, et en présence d'un polysaccharide. Ces particules peuvent immobiliser des anticorps, des oligonucléotides, des lectines ou d'autres biomolécules, par couplage sur le polysaccharide grâce à des méthodes de greffage connues. Leur utilisation a souvent été reprise, comme dans les documents US-C-5,543,289 ou WO-A-88/00060, ou elles sont utilisées dans des applications particulières comme celles décrites dans les documents FR-A-2 710 410 et FR-A-2 732 116. Le document US-C-4,795,698 décrit une modification de la procédure de Molday, en remplaçant, par exemple, le polysaccharide par un autre polymère de nature protéique. Les protéines présentes à la surface des particules peuvent ainsi servir a l'immobilisation ultérieure d'anticorps par des méthodes de couplage connues de l'homme de l'art.

Ces particules nécessitent l'utilisation de montages particuliers permettant d'augmenter localement le gradient de champ magnétique. Cette technique est connue par l'homme de l'art sous l'appellation de HGMS (pour High Gradient Magnetic Separation) et est par exemple décrite par WO 96/09409. Elle utilise un dispositif consistant, par exemple, en un récipient contenant une matrice de fibres ou de grains métalliques. Placé dans un champ magnétique externe, ce dispositif permet d'obtenir un gradient de champ très important à la surface même des fibres. Même des particules superparamagnétiques de dimensions restreintes peuvent être retenues sur un tel dispositif, pour peu que le réseau de fibres soit suffisamment resserré et que le trajet d'une particule l'amène au voisinage de la matrice. A titre d'exemple, de tels dispositifs sont décrits dans les documents US-C-4,375,407, US-C-5,543,289, WO-A-96/26782, WO-A-96/26011 ou US-C-5,186,827, et des publications comme celle de B.L. Hirschbein et al. (CHEMTECH, March 1982, pp 172-179). Des montages peuvent également être trouvés dans le commerce, par exemple, sous l'appellation MACS column chez Miltenyi Biotec (Bergisch Gladbach, Germany).

Ces méthodes sont répandues et même commercialisées pour la séparation et la concentration de cellules biologiques de leurs milieux aqueux. En fait, dans de telles applications, plusieurs milliers de particules peuvent réagir avec une seule cellule par l'intermédiaire de réactions de reconnaissance moléculaire spécifiques, ce qui permet de séparer cet élément en augmentant sa masse magnétique. En revanche, en raison de leur faibles dimensions, les particules ne peuvent réagir spécifiquement qu'avec un nombre très réduit de molécules dispersées en solution. Elles restent donc sous forme discrète et pour cette raison, ne sont pas utilisées pour la séparation, la concentration ou l'enrichissement d'un analyte.

De plus, après séparation, les particules sont plutôt considérées comme une gêne dans les étapes ultérieures de tout procédé. Dans le document US-C-4,018,886, elles sont même éliminées volontairement. Le document FR-A-2 710 410 utilise la présence de particules superparamagnétiques de faible taille comme éléments de détection permettant de quantifier une réaction de reconnaissance moléculaire. Dans ce cas, le principe utilisé est une réaction d'agglutination résultant en la formation d'un agrégat. De plus, les particules ne sont pas utilisées comme moyen de concentration ou de séparation de l'élément à séparer, mais uniquement comme moyen de détection.

En résumé, les particules magnétiques relativement petites sont peu adaptées à des processus de séparation, enrichissement ou concentration, dans des processus traditionnels, notamment immuno-essais.

Le document WO-A-9609409 décrit l'utilisation du procédé dit MACS (Magnetic Cell Sorting), impliquant la formation d'un complexe entre un analyte et un réactif comprenant des particules magnétiques, pour enrichir/concentrer ledit analyte, en l'occurrence des érythrocytes foetaux. Ces cellules sont ensuite analysées par cytométrie de flux, ou utilisées comme source de matériel génétique, mais sans être immobilisées sur un support, par une liaison entre l'analyte et un récepteur par exemple.

Le document DE-A-4 036 288 décrit un procédé permettant de détecter et quantifier un ou plusieurs analytes liés spécifiquement à des particules susceptibles d'être distinguées.

La présente invention a pour objet un procédé permettant d'isoler un analyte à partir d'un milieu liquide dans lequel il est extrêmement dilué ou peu concentré. Plus particulièrement, s'agissant de la détermination de l'analyte, la présente invention a pour objet un procédé permettant une grande sensibilité de détection et/ou de quantification.

Conformément à la présente invention, de manière générale :
- on met en contact le réactif avec un échantillon liquide obtenu à partir du milieu contenant l'analyte, pour former un milieu intermédiaire comprenant, toujours sous forme discrète et distribuée dans ledit milieu, un complexe entre ledit support, le récepteur et l'analyte,
- on met en contact la surface accessible dudit autre support avec au moins un partenaire de capture, à savoir le réactif n'ayant pas réagi lors de l'étape précédente, ou le complexe, pour lier ledit partenaire de capture à ladite surface accessible, et obtenir un dépôt concentré dans ladite zone utile, immobilisé sur ledit autre support par la liaison dudit autre récepteur avec ledit récepteur ou l'analyte,
- on concentre le dépôt dudit partenaire de capture dans ladite zone utile, en augmentant la quantité dudit partenaire par unité de surface dans ladite zone utile.

Plusieurs modes de concentration du dépôt peuvent être considérés, avant ou lors de la capture en contact avec la surface accessible de l'autre support.

On concentre le dépôt du partenaire de capture, en obtenant à partir du milieu intermédiaire un autre échantillon, enrichi en partenaire de capture ; et ledit autre échantillon est mis en contact avec la surface accessible.

On établit un flux liquide, contenant le partenaire de capture, de section limitée et adaptée à la surface accessible, et ledit flux est mis en contact avec ladite surface accessible.

On établit un flux liquide, contenant le partenaire de capture, et ledit flux est mis en contact avec ladite surface accessible, puis recyclé au contact de cette dernière.

Avant la description de l'invention, il convient de donner une définition des termes utilisés.

On entend par "ligand", un élément capable de former par un lien chimique ou physique, un complexe avec un analyte.

A titre d'exemple de ligand, on peut citer les anticorps, les fragments d'anticorps, les antigènes, les haptènes, les lectines, les sucres, les acides ribo- et désoxyribonucléiques, les protéines notamment A ou G, les hormones, les récepteurs d'hormones, la biotine, l'avidine ou la streptavidine et, d'une manière générale, les ligands naturels ou synthétiques, et les analogues de ligands modifiés, pouvant entrer en compétition avec les ligands.

On entend par "récepteur", tout ligand tel que précédemment défini, immobilisé sur un support par un moyen quelconque tel qu'adsorption, covalence, chélation, reconnaissance moléculaire, et capable de retenir un analyte, seul ou conjugué à un autre ligand.

on entend par "support", tout type de support, polymère, inorganique ou métallique. A titre d'exemple de supports polymères, on peut citer les supports plastiques à base de polystyrène, poly(méth)acrylates, polybutadiène, polypropylène, ou d'autres, seuls ou sous forme de copolymères. A titre d'exemple de supports inorganiques, on peut citer l'oxyde de silice, le silicium, le mica, le verre, le quartz. A titre d'exemple de supports métalliques, on peut citer l'or, l'argent, l'oxyde de titane, l'oxyde de vanadium.

L'immobilisation des ligands sur le support peut s'effectuer, soit par simple adsorption sur le support natif ou modifié, soit par l'intermédiaire d'une réaction chimique ou physique permettant de modifier la surface du support, et ainsi de permettre la fixation du récepteur par des liens de covalence, ou d'autres moyens traditionnels bien connus de l'homme de l'art.

On entend par "surface limitée", toute surface obtenue par des moyens chimiques ou physiques, visant à réduire une surface de dimensions données, par exemple par découpe en éléments de taille inférieure, par recouvrement par un "masque" limitant la surface initiale aux contours internes du masque, ou par des moyens chimiques d'étalement selon une surface moindre, comme décrit dans Kumar A. et al (Langmuir (1994), 10, 1498-1511). La réduction de la surface de la zone utile ou active n'est pas limitée en taille. Des procédés issus des microtechnologies permettent, par exemple, d'obtenir des surfaces limitées de dimensions micro ou nanoscopiques, ainsi que de déposer et/ou d'acheminer des micro ou nanovolumes de liquides vers ladite zone utile de surface limitée.

Dans la description ci-après, le terme "conjugué" sera réservé à l'entité formée par immobilisation entre le ligand et le support du réactif. Le terme "complexe" sera réservé à l'entité formée entre le réactif, par hypothèse conjugué, et l'analyte. Qu'il s'agisse du support faisant partie du réactif, du réactif proprement dit, et du complexe, ceux-ci sont sous forme discrète, distribuée ou dispersée au sein d'un milieu liquide, et par conséquent sous forme de particules.

Les termes "support" et "autre support" indiquent qu'il s'agit d'éléments ayant substantiellement la même nature ou la même fonction. Le terme "support" sera réservé au support appartenant au réactif, et le terme "autre support", au support dont au moins une partie de la surface, ou fenêtre, accessible, comporte une ou plusieurs zones utiles de surface limitée.

Les termes "récepteur" et "autre récepteur" seront réservés respectivement aux ligands immobilisés respectivement sur ledit support et ledit autre support.

On entend par "particule", toute particule d'un support polymère, inorganique pu métallique, sur laquelle il est possible de greffer un ligand. En particulier, sont considérées comme tombant dans le champ de la présente invention, les particules pouvant être séparées par l'action d'un moyen physique extérieur, par exemple par voie magnétique ou électrique, ou sous l'effet de la gravité ou par centrifugation. Ressortent de la définition précédente, les particules de faible taille, notamment superparamagnétiques, dont la vitesse de sédimentation sous l'effet de la gravité est inférieure à l'agitation thermique, mais pouvant constituer des agrégats par tout procédé permettant de les réunir entre elles, ou de les assembler sur des particules de plus grosses tailles, séparables par un moyen physique quelconque.

A titre d'exemple de particules polymères, on peut citer les particules obtenues par polymérisation en émulsion telles que les latex, ou des particules de plus grosse taille, magnétiques ou non.

A titre d'exemple de particules métalliques, on peut citer l'or colloïdal, les particules ferro-, ferri-, para- ou superparamagnétiques, recouvertes ou non de polymères naturels ou synthétiques, dont la composition comprend le fer ou d'autres métaux comme le cobalt, le nickel, seuls ou sous forme d'alliages, magnétiques ou non.

A titre d'exemple de particules inorganiques, on peut citer les particules à base de silice ou de silicium, magnétiques ou non.

A titre d'exemple de méthodes de séparation par un moyen physique extérieur, on peut citer la sédimentation par gravité ou par centrifugation, l'attraction magnétique par l'action d'aimants permanents, d'électro-aimants, ou par l'utilisation de dispositifs permettant d'augmenter le gradient de champ magnétique, l'attraction électrique, et toute autre technique équivalente.

On entend par "détermination", toute méthode permettant de mettre en évidence la présence et/ou de quantifier le dépôt concentré dans la zone utile de la surface accessible dudit autre support, à savoir les particules du produit spécifique d'intérêt, c'est-à-dire du complexe ou du réactif n'ayant pas réagi.

A titre d'exemple de méthode de détermination, on peut citer :
- des méthodes topographiques telles que la microscopie à force atomique, profilométrie, ...
- des méthodes magnétiques telles que décrites par exemple dans le document FR-A-2 710 410, la microscopie à force magnétique, et tous procédés de détermination sensibles à la présence d'éléments métalliques ferro, ferri, para et super paramagnétiques
- des méthodes électriques telles que la mesure d'une variation de capacité, décrite par exemple dans le document EP-A-90 402 611, la microscopie à effet tunnel, les mesures d'impédance, ...
- des méthodes optiques permettant la mise en évidence, par exemple, d'une modification de l'épaisseur et/ou de l'indice de réfraction (couches minces optiques, ellipsométrie, résonance de plasmons de surface, ondes acoustiques de surface,...), ou la mesure d'une intensité lumineuse (ondes évanescentes, microscopie à fond noir, microscopie en champ proche optique, ...)
- des méthodes permettant la mesure de variations de masses (microbalance à cristal de quartz, ...)
- et d'une manière générale, toutes techniques non citées ici, mais équivalentes.

La présente invention peut recevoir différents modes d'exécution, en fonction des choix opératoires définis ci-après.

Dans l'étape de concentration, le taux de concentration en partenaire de capture est déterminé pour que la quantité dudit partenaire de capture immobilisé demeure au plus égale à la quantité dudit autre récepteur sur ledit autre support. A cette fin, par exemple, le réactif comprend des particules du support conjugué avec le récepteur, ayant la capacité d'être séparées de tout milieu liquide dans lequel elles sont dispersées, sous l'action d'un moyen physique appliqué au milieu liquide, et on applique ce moyen physique à au moins une partie du milieu intermédiaire, pour séparer les particules complexes, on lave éventuellement ces particules complexes ainsi séparées, et on récupère les particules complexes, en cessant d'appliquer le moyen physique, pour obtenir ultérieurement dans ladite zone utile un dépôt concentré en partenaire de capture. A titre d'exemple, les particules du support sont magnétiques, en particulier superparamagnétiques, et le moyen physique consiste en un champ magnétique appliqué au milieu intermédiaire, par exemple selon la technique dite HGMS (High Gradient Magnetic Separation).

Le récepteur, c'est-à-dire celui appartenant au réactif, et ledit autre récepteur, c'est-à-dire celui de la surface accessible de capture, comportent le même ligand, immobilisé respectivement sur le support et ledit autre support.

Lorsque le réactif comprend des particules du support conjugué avec le récepteur, ayant la capacité d'être séparées de tout milieu liquide dans lequel elles sont dispersées, sous l'action d'un moyen physique appliqué à ce milieu liquide, préférentiellement on applique ce moyen physique en relation avec l'autre support, c'est-à-dire celui de capture, pour concentrer ledit dépôt dans toute zone utile.

Dans certains cas, le réactif est marqué, et le traitement de l'analyte comprend une étape de détermination du marqueur du dépôt concentré, immobilisé dans la zone utile de l'autre support, c'est-à-dire du support de capture. Classiquement ce marquage du réactif est obtenu selon l'un des modes suivants, à savoir:
- le support constitue lui-même un marqueur ; il s'agit par exemple d'or colloïdal
- le marqueur est lié au support
- le marqueur est lié au récepteur.

On détermine l'analyte à partir du dépôt concentré dans la zone utile, par toute méthode choisie dans le groupe incluant les méthodes topographiques, par exemple par microscopie à force atomique (AFM), les méthodes magnétiques, par exemple par microscopie à force magnétique, les méthodes électriques, par exemple mesure d'une variation de capacité, les méthodes optiques, par exemple mesure de l'indice de réfraction, et enfin les méthodes de mesure de variation de masse, par exemple microbalance à cristal de quartz.

Plus particulièrement, la zone utile est dimensionnée pour que la quantité dudit dépôt immobilisé sur ledit autre support soit au moins égale au seuil de sensibilité de la méthode de détermination, exprimée en nombre de particules par unité de surface.

Le format d'essai utilisé est soit direct, auquel cas ledit partenaire de capture est ledit complexe entre ledit support, le récepteur et l'analyte, soit par compétition, auquel cas ledit partenaire de capture est ledit réactif.

Préférentiellement, on obtient un autre échantillon, en enrichissant ledit milieu intermédiaire en complexe, et ledit autre échantillon est mis en contact avec la surface accessible dudit autre support.

Différents modes de mise en contact de l'autre échantillon avec la surface accessible de capture sont considérés:
- on dépose une quantité dosée dudit autre échantillon sur la surface accessible de ce dernier
- ou on fait passer un courant dudit autre échantillon, d'épaisseur relativement faible, éventuellement recyclé, au contact de cette surface accessible.

Enfin, le milieu liquide peut contenir une pluralité d'analytes différents, et alors la surface accessible de capture de l'autre support comprend une pluralité de zones utiles, exhibant une pluralité d'autres récepteurs respectivement différents, de partenaires de capture respectivement différents.

Le procédé selon l'invention peut être appliqué à un matériel nucléique particulier de toute bactérie, en l'occurrence de l'ARN ribosomique, auquel cas le niveau de sensibilité obtenu permet d'éviter toute méthode d'amplification enzymatique, telle que PCR (Polymerase Chain Reaction), appliquée au matériel génomique de la même bactérie, pour détecter cette dernière. En effet, le niveau de sensibilité obtenu devient compatible avec la quantité d'ARN ribosomique présente dans une bactérie, de l'ordre de 10⁴ à 10⁵ molécules, ce qui permet de déterminer la présence de cette bactérie, sans recourir à une amplification de tout ou partie de son génome.

### DESCRIPTION DES FIGURES

La figure 1 représente un schéma général de la formation du complexe en solution.

La figure 2 montre un exemple de dispositif de séparation du complexe.

La figure 3 représente une surface accessible, biospécifique du support de capture. La figure 4 représente le dépôt de la solution contenant le complexe sur la surface biospécifique. La figure 5 représente la réaction entre le complexe et les autres récepteurs de la surface biospécifique.

Les figures 6 et 6bis présentent de manière schématique deux méthodes permettant de rassembler et immobiliser des particules du complexe sur la surface biospécifique, l'une sans concentration du complexe (Fig. 6), et l'autre avec concentration du complexe (Fig. 6bis).

La figure 7 représente deux courbes issues des méthodes présentées sur les figures 6 et 6bis respectivement. En ordonnées, est porté le nombre de particules détectées par unité de surface, et en abscisse la concentration du milieu liquide d'origine en analyte. Le trait en pointillé établit le seuil de sensibilité.

La figure 8 montre une surface accessible au complexe, appartenant audit autre support, dit de capture, limitée par l'utilisation d'un masque.

Les figures 9 et 9bis présentent le dépôt de particules du complexe, sur la surface limitée représentée sur la figure 8, respectivement sans et avec un moyen de concentration, constitué par un aimant ou électro-aimant.

La figure 10 représente un système identique à celui représenté sur la figure 4, avec la surface limitée représentée figure 8 et l'adjonction d'un aimant.

La figure 11 représente l'effet de l'enrichissement en complexe par un gradient de champ magnétique, sur la variation du nombre de particules par unité de surface (µm²) en ordonnées, en fonction de la concentration en TSH (analyte) en abscisses [Log10 ng/ml]. Les ronds noirs réfèrent à une absence d'enrichissement avec un volume constant de 0,07 ml, les carrés noirs à un volume variable concentré par HGMS.

La figure 12 montre quatre images de microscopie à force atomique qui représentent l'effet de l'addition d'un aimant pendant la réaction de formation des conjugués entre complexes et récepteurs de la surface biospécifique, ou accessible du support de capture. Les figures de gauche sont obtenues avec concentration par un aimant, et les figures de droite sans concentration.

La figure 13 montre l'évolution du nombre de particules immobilisées du complexe par unité de surface (µm²), en fonction de la taille de la surface biospécifique en mm² (en abscisses).

La figure 14 montre l'évolution du nombre de particules immobilisées du complexe par unité de surface (µm²), en fonction de la concentration d'origine en TSH en solution en pg/ml en abscisses, pour une surface biospécifique de faibles dimensions; la figure de droite (Fig. 14bis) montre la pente d'origine.

La figure 15 compare l'évolution du nombre de particules immobilisées du complexe par unité de surface (µm²), en fonction de la concentration d'origine en TSH en solution, en ng/ml en abscisses, selon la dimension de la surface biospécifique. Les ronds noirs réfèrent à un complexe, d'abord enrichi par gradient de champ magnétique HGMS, immobilisé sans concentration sur une surface biospécifique de 64 mm². Les croix noires réfèrent à un complexe enrichi, et immobilisé avec concentration sur une surface biospécifique de 0,5 mm². La concentration en abscisses est en 10g10 (ng/ml).

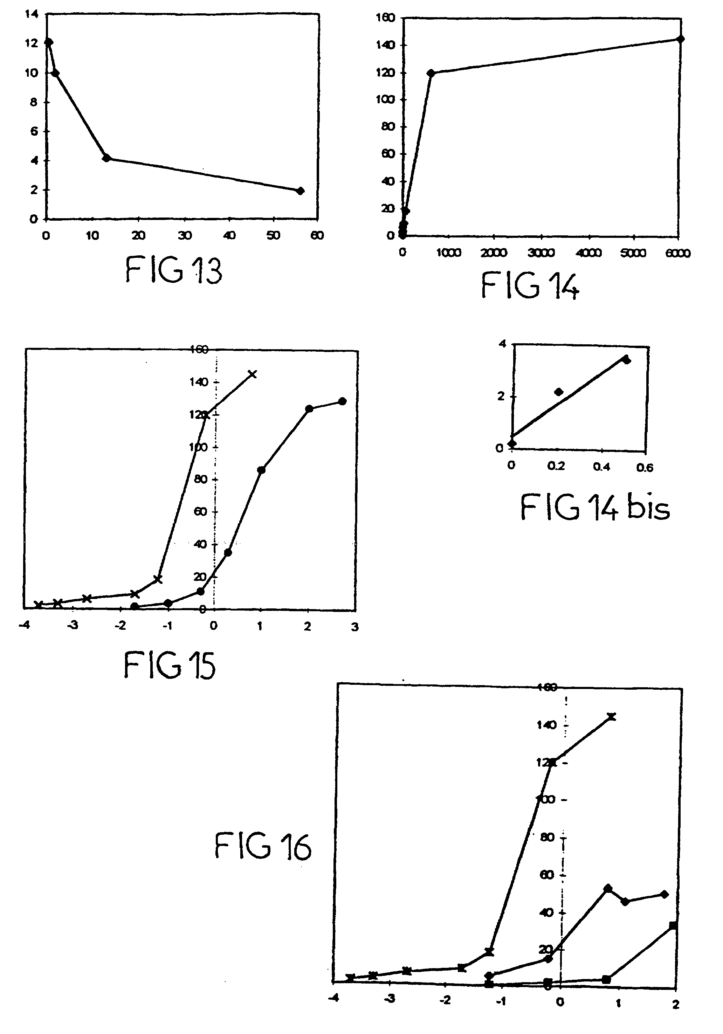

La figure 16 représente une comparaison des différents modes de concentration en utilisant des particules magnétiques pour le réactif de départ. Les ordonnées et abscisses sont les mêmes, avec les mêmes échelles, que celles représentées à la figure 15. Les carrés noirs réfèrent à un complexe immobilisé sur la surface biospécifique, sans enrichissement préalable, ni concentration au moment de la capture sur la surface biospécifique, les losanges noirs à un complexe immobilisé après enrichissement sur colonne HGMS, et les croix noires à un complexe immobilisé, après enrichissement et avec concentration.

La figure 17 montre une comparaison des différents modes d'enrichissement, en utilisant des particules d'or colloïdal à titre de support appartenant au réactif. En ordonnées, est porté le nombre de particules par µm², en abscisses la concentration en THS (Log 10 ng/ml). Les losanges noirs réfèrent à une détermination après enrichissement par centrifugation, et les ronds noirs à une détermination sans enrichissement par centrifugation.

La figure 18 représente un mode d'exécution de l'invention pour le dosage d'haptènes par compétition et la figure 19 la courbe théorique attendue, exprimant en ordonnées le nombre de particules détectées par unité de surface biospécifique, et en abscisses la concentration d'origine en haptène.

La figure 20 représente une surface biospécifique accessible du support de capture, comportant plusieurs zones utiles avec des récepteurs d'affinités respectivement différentes.

La figure 21 représente un autre mode d'exécution de l'invention.

La figure 22 représente un autre mode d'exécution de l'invention.

Les figures 23 et 24 représentent le mode d'exécution décrit sur la figure 22 auquel il a été ajouté deux moyens de concentration, et une surface biospécifique comportant plusieurs zones utiles telle que décrite figure 20, par exemple.

Les figures 25 et 26 représentent un autre mode d'exécution de l'invention. Elles présentent notamment l'adjonction à la surface de l'aimant 12, d'une pièce pointue 21 en matériau magnétique dit "mou" possédant la propriété de confiner les lignes de champ de l'aimant à l'extrémité de cette pointe. Cette pièce ou l'équivalent peut aussi bien être ajoutée aux exemples décrits sur les figures 9bis, 10, 18, 21, 23 et 24 ou tout autre montage équivalent.

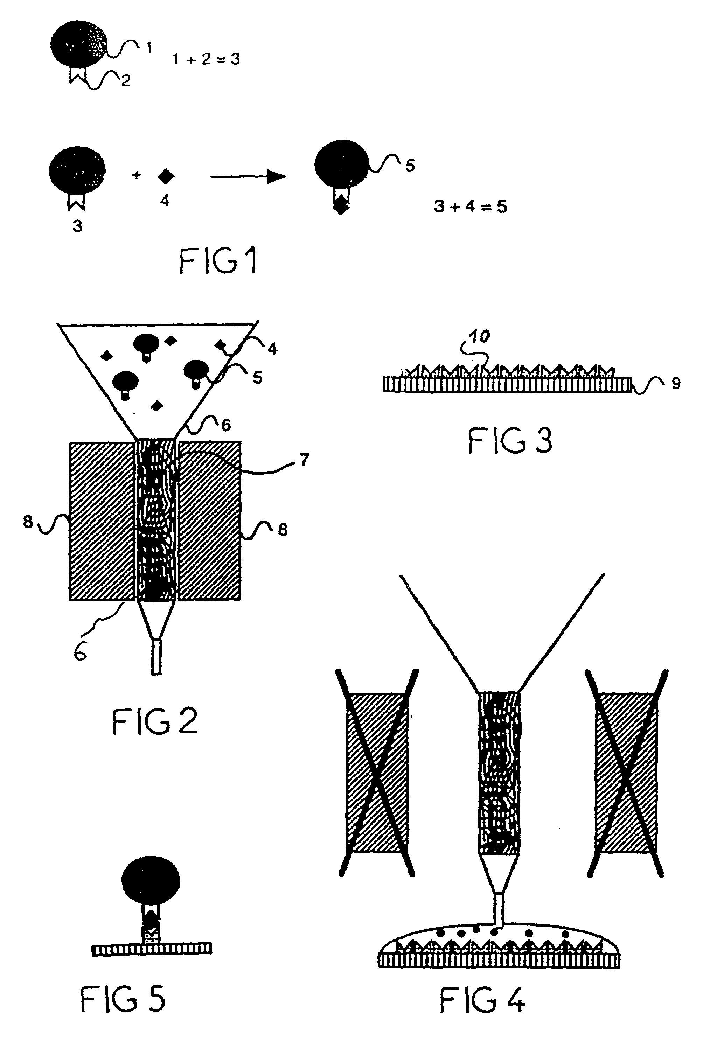

Comme il en a été fait mention précédemment, l'invention a, comme aspect particulier, un procédé permettant d'isoler des analytes, même très dilués, de milieux liquides. Le mode d'isolement peut être variable, un exemple indicatif est donné afin de faciliter la, compréhension. Ce mode présente l'avantage d'utiliser des particules magnétiques de faible taille. La figure 1 en montre un exemple.

Dans un mode de mise en oeuvre particulier, les particules 1 utilisées ont un diamètre compris entre 10 et 1000 nm, préférentiellement compris entre 10 et 200 nm. Elles comprennent au moins un noyau d'oxyde de fer monocristallin, de diamètre compris entre 7 et 15 nm, entouré lui-même d'une couche d'un polymère hydrophile (support), de manière à éviter le contact entre le noyau métallique et le ligand formant récepteur. Le noyau métallique, compte tenu de sa dimension possède un caractère superparamagnétique. De telles suspensions sont stables plusieurs mois, sans sédimentation notable. Le polymère peut être avantageusement utilisé pour l'immobilisation ou greffage des ligands selon des méthodes connues de l'homme de l'art. Le polymère utilisé ici est un polysaccharide appelé Dextran®, de masse molaire comprise entre 10,000 et 2,000,000, préférentiellement comprise entre 40,000 et 70,000. Il peut être opportunément activé ou fonctionnalisé par différentes méthodes telles qu'oxydation par le periodate, donnant des fonctions aldéhyde par coupure entre fonctions alcool vicinales, ou par le bromure de cyanogène, et d'une manière générale, par toute méthode équivalente.

Les ligands 2 sont indifféremment, des anticorps, des antigènes, des oligonucléotides, ou tout autre entité biologique présentant la propriété de se lier à un analyte, ainsi qu'il a été défini ci-dessus. Les ligands peuvent, par exemple, être couplés à la surface des particules, sur les fonctions du polymère précédemment activé, par l'intermédiaire de fonctions amine primaire existant dans la molécule native ou ajoutées intentionnellement au ligand. Le conjugué particule/ligand 3 est représenté figure 1.

La figure 1 représente un schéma général de la formation des complexes en solution. Le conjugué 3 (réactif) est introduit dans le milieu contenant l'analyte 4 à séparer. Après un temps d'incubation nécessaire à la formation du complexe 5 entre conjugué et analyte, la solution (milieu intermédiaire) est passée sur le montage représenté sur la figure 2. Il s'agit d'un exemple de dispositif d'enrichissement basé sur le principe d'une séparation d'éléments répondant à la sollicitation d'un champ magnétique. Il comporte une chambre de séparation 6 abritant une matrice de filaments en matériau ferromagnétique, tel que filaments d'acier 7. Cette matrice peut-être opportunément recouverte d'un polymère, tel que décrit dans les documents US-C-4,375,407 ou WO-A-90/07380. La solution contenant les complexes analyte/ réactif 5, est passée sur la matrice filamenteuse placée dans le champ magnétique d'un ou plusieurs aimants 8, à un débit ajustable et permettant au complexe d'être retenu par la matrice. Un tel dispositif permet d'enrichir le complexe dilué dans des volumes initiaux très importants.

Après élimination des éléments étrangers et du liquide porteur, le champ magnétique est retiré, et le complexe concentré est élué par un faible volume de liquide. Ce volume est déposé sur une surface accessible, biospécifique, représentée sur la figure 3. Elle est constituée d'un support plan 9 sur lequel sont immobilisés d'autres récepteurs 10 spécifiques de l'analyte 4.

Un support plan tel que l'oxyde de silicium présente des avantages connus. On peut citer par exemple, une rugosité faible, des dimensions ajustables ou des modes de fonctionalisation variés. Par exemple, la fixation d'alkoxy ou de chlorosilanes permet l'introduction de fonctions en surface, dans le but d'immobiliser des récepteurs de différentes façons (adsorption, liaisons covalentes, ...),... D'autres types de supports tels que décrits précédemment sont cependant utilisables.

La figure 4 représente un exemple de dépôt de la solution contenant les complexes 5. Celle-ci est éluée en retirant le champ magnétique et est éluée et déposée sur la surface biospécifique dans un volume faible.

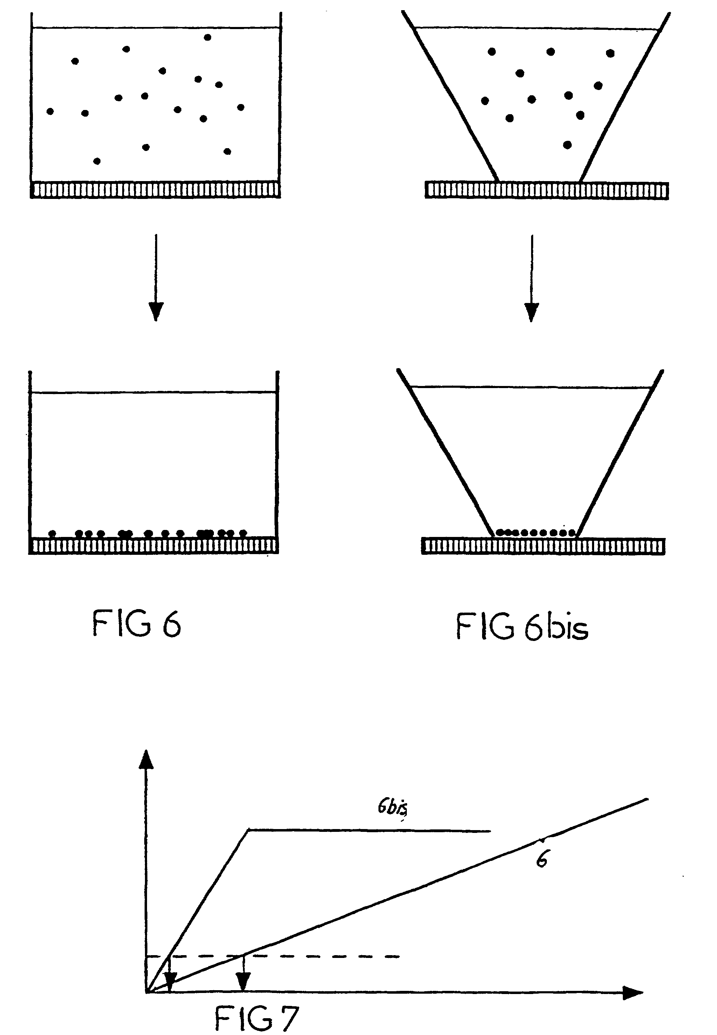

La figure 5 représente un exemple de la réaction entre le complexe 5 et le récepteur 10 sur le support 9.

Les figures 6 et 6bis présentent l'un des avantages de l'invention. Sur la figure 6, un nombre donné de particules est concentré sur une surface biospécifique, dite large. La figure 6bis présente une amélioration, en ce que le même nombre de particules est concentré sur une surface biospécifique de dimensions plus faibles que sur le montage de la figure 6.

La figure 7 représente une courbe théorique montrant l'augmentation du nombre de particules par unité de surface, selon les montages représentés sur les figures 6 et 6bis. Si une limite de sensibilité (traits pointillés) est représentée, il est avantageux d'utiliser une surface réduite, en ce que cela permet une amélioration du seuil de sensibilité et donc, la détection de concentrations de particules en solution plus faibles.

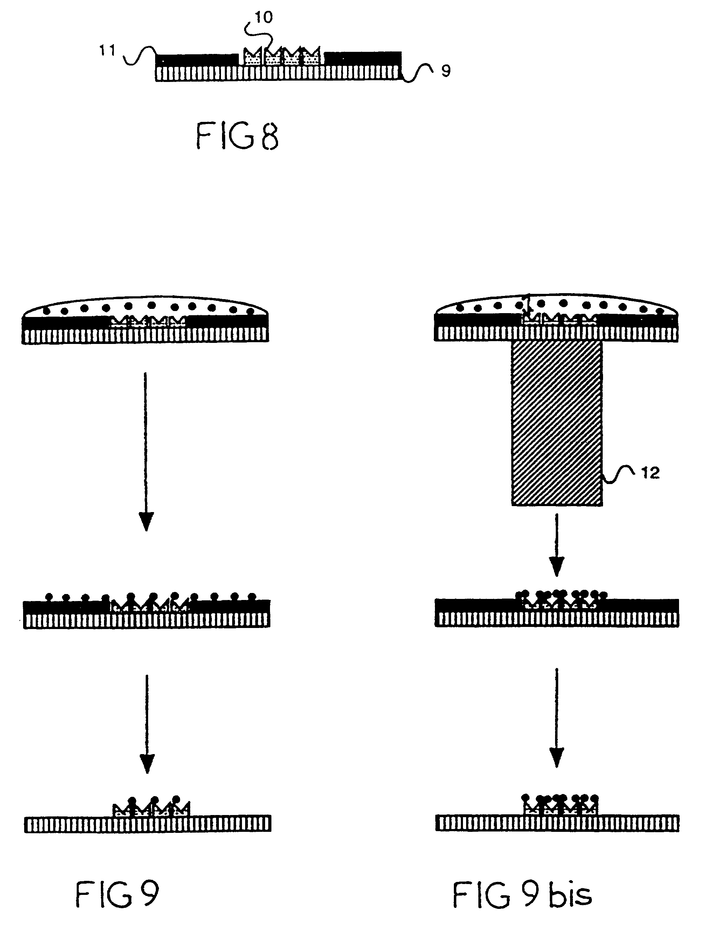

La figure 8 montre un exemple de mise en oeuvre visant à limiter la surface biospécifique constituée par une seule zone utile de capture. Celle-ci est identique à la surface biospécifique représentée sur la figure 4, mais sa dimension a été limitée par l'utilisation d'un masque 11 de dimension contrôlée.

Conformément à la description précédente, les figures 9 et 9bis présentent une mise en oeuvre particulière du dépôt de particules sur la surface réduite représentée sur la figure 8. La figure 9bis présente un autre avantage de l'invention. Il s'agit d'une amélioration du mode représenté sur la figure 9, par l'adjonction d'un aimant 12 placé sous le support et visant à concentrer les complexes 5 sur la zone utile 10.

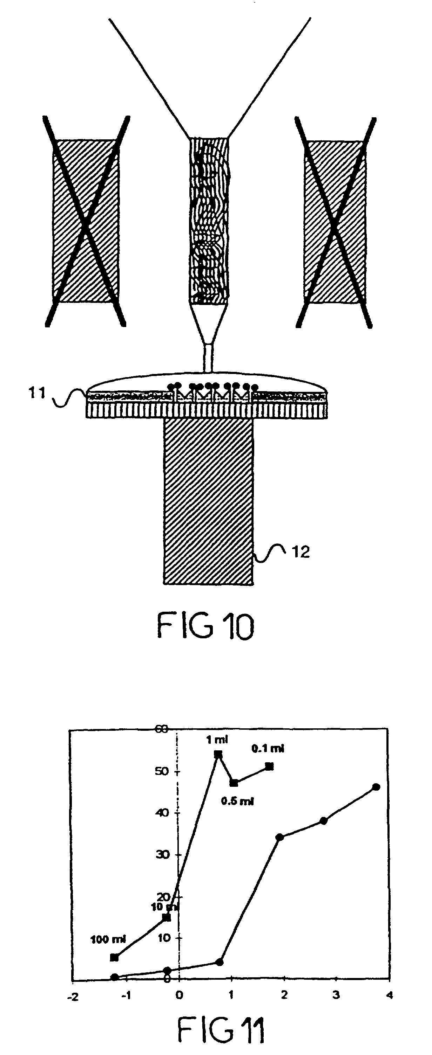

La figure 10 représente un système identique à celui représenté sur la figure 4, avec les deux perfectionnements de l'invention décrits ci-dessus: la réduction de la surface accessible par l'adjonction d'un masque 11, et la concentration ou confinement des particules sur une surface de dimensions réduites par l'adjonction d'un aimant 12.

Dans une mise en oeuvre particulière à l'invention, les particules sont détectées et quantifiées par microscopie à force atomique. Cette technique permet, non seulement de détecter et d'identifier les particules sans ambiguïté, mais aussi, d'utiliser des logiciels de traitement d'images pour la quantification du nombre de particules présentes sur une surface définie.

Après cette étape de concentration, la réaction est détectée ou quantifiée par microscopie à force atomique, mais d'autres moyens connus de l'homme de l'art peuvent être employés et font partie du domaine de l'invention.

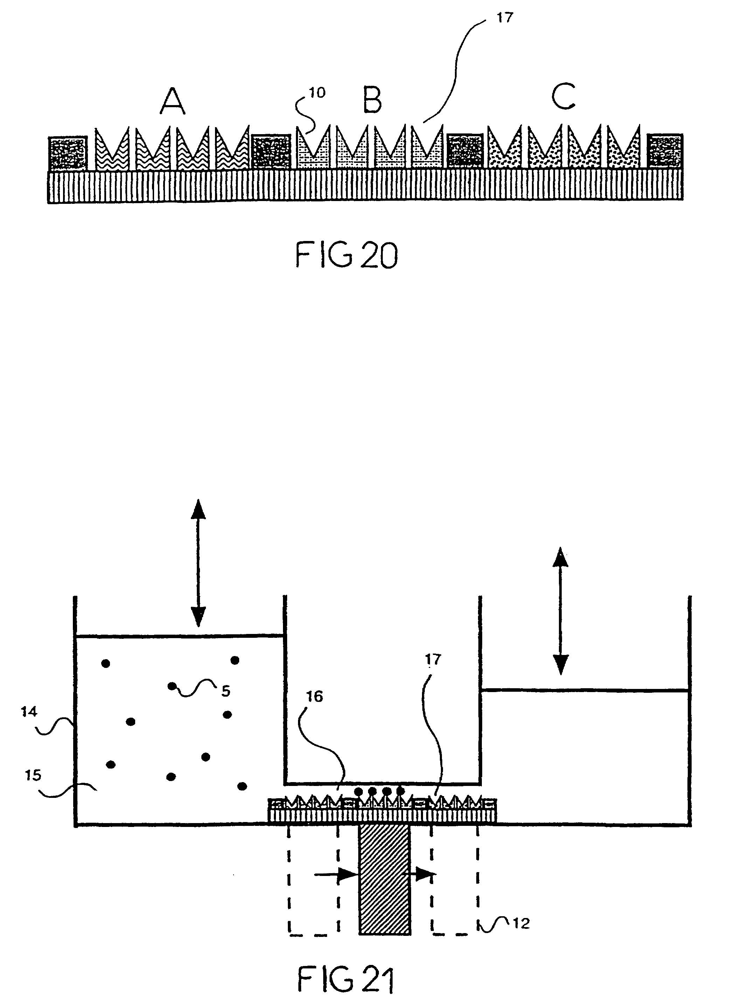

La figure 20 montre un exemple de mise en oeuvre visant à limiter plusieurs surfaces biospécifiques ou zones utiles de capture (A, B, C ...), chacune de spécificité différente. La dimension de chacune a été limitée par l'utilisation d'un masque 11 de dimension contrôlée.

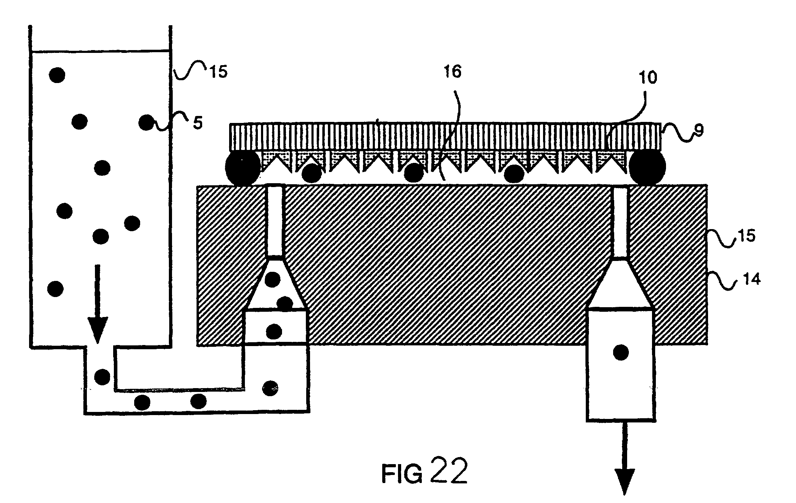

Dans un mode particulier d'exécution de l'invention, la figure 22 représente un moyen de séparation des analytes 4 ou des complexes 5 de la solution. Une cellule de hauteur limitée et comportant une entrée et une sortie permet une mise en contact de l'échantillon contenant les analytes 4 ou les complexes 5 avec les récepteurs 10 de la surface de capture. Le débit est ajustable par tout moyen connu de l'homme de l'art, et la géométrie de la cellule peut être adaptée à un écoulement laminaire ou turbulent.

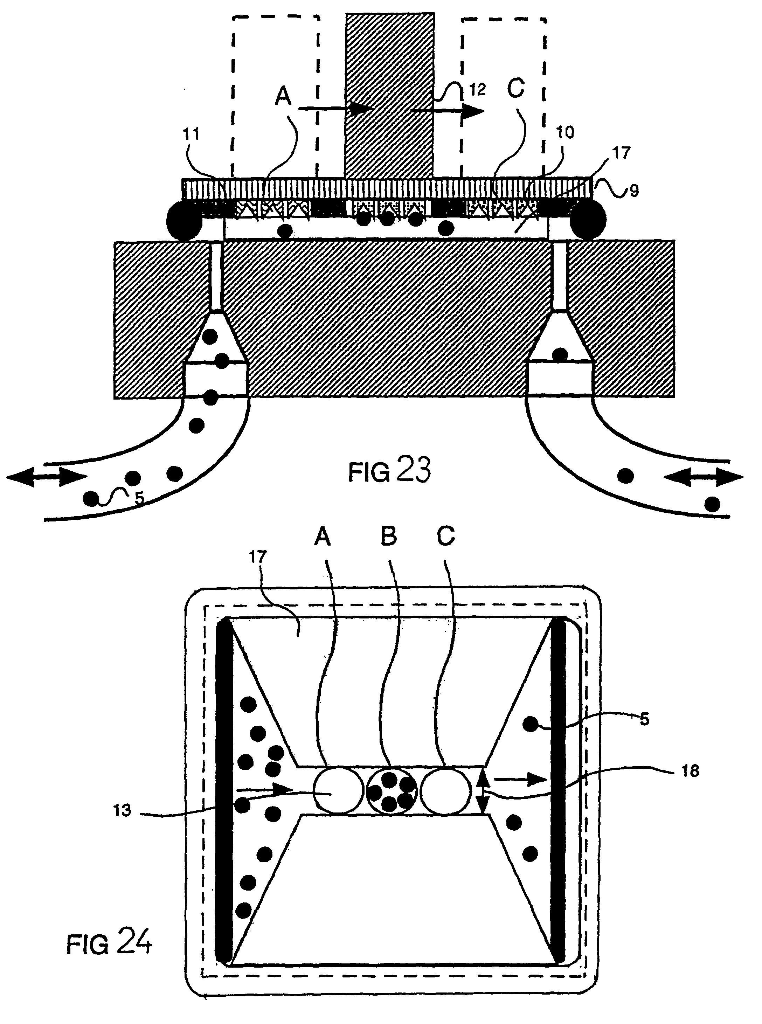

Les figures 23 et 24 représentent respectivement une coupe de profil et une vue de dessus du mode d'exécution décrit sur la figure 22, auquel il a été ajouté deux moyens de concentration et une surface biospécifique comportant plusieurs zones utiles avec des récepteurs d'affinités respectivement différentes, telle que décrite à la figure 20. Le premier moyen de confinement est, par exemple, l'emploi de "cales" 17 visant à confiner le flux de la solution à un chemin de largeur 18 passant au contact des zones actives 13 A, B et C de la surface de capture. Le deuxième moyen de confinement est l'adjonction d'un aimant mobile 12 ou de plusieurs aimants en regard de chaque zone utile.

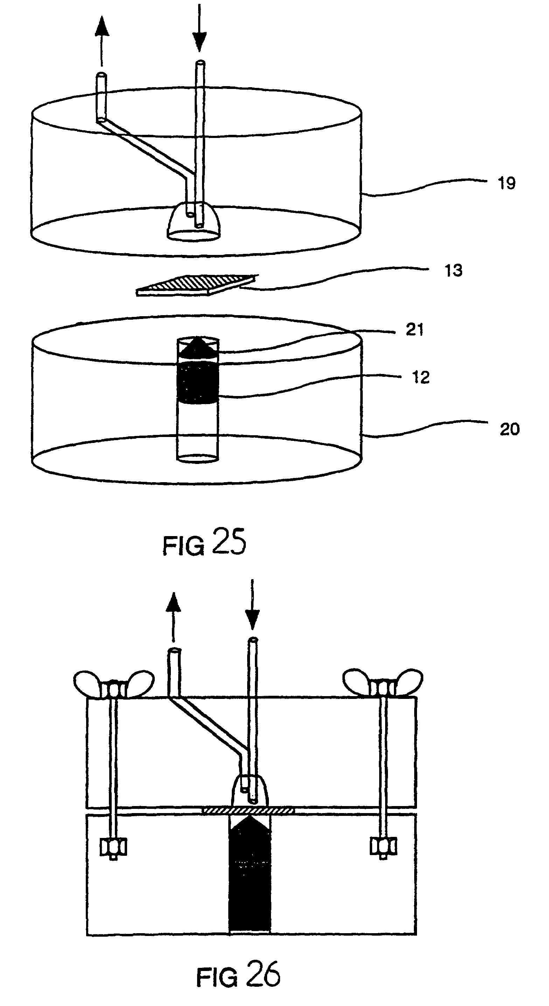

Les figures 25 et 26 représentent un autre mode d'exécution de l'invention. Elles présentent en particulier, dans la partie inférieure 20, l'adjonction à la surface de l'aimant 12, d'une pièce pointue 21 d'un matériau magnétique dit "mou" possédant la propriété de conduire et de confiner les lignes de champ de l'aimant à l'extrémité de cette pointe. Cette pièce ou l'équivalent peut aussi bien être ajoutée aux réalisations selon les exemples décrits sur les figures 9bis, 10, 18, 21, 23 et 24, ou tout autre montage équivalent. La partie supérieure 19 est munie d'une entrée et d'une sortie permettant une mise en contact des analytes 4 ou des complexes 5 de l'échantillon avec les récepteurs 10 de la surface de capture 13.

Des détails additionnels concernant la mise en oeuvre de l'invention sont indiqués dans les exemples suivants.

### EXEMPLES :

### Exemple 1- Fabrication des surfaces limitées, biospécifiques, par couplage d'anticorps anti-TSH (Thyroid Stimulating Hormon, ou analyte)

Le support utilisé est composé de plaquettes de silicium de 8 x 8 mm, recouvertes de silice thermique. Les éventuelles contaminations organiques sont d'abord éliminées par nettoyage dans le mélange sulfochromique. Les plaquettes sont ensuite immergées dans 1 ml d'une solution d'aminopropyldiméthyléthoxysilane à 2% (v/v) dans le toluène. Les fonctions amine ainsi créées sur la surface sont activées par 1 ml de solution de disuccinimidyl subérate (10 mM dans le DMF).

Les anticorps anti-TSH de capture sont dilués à 40 µg/ml en tampon phosphate 50 mM, NaCl 0,15 M, pH 7,4 (PBS). 70 µl sont déposés sur les plaquettes activées. Le couplage a lieu pendant 1 heure à température ambiante. Les plaquettes sont ensuite incubées pendant 2 heures à 37°C avec 70 µl d'une solution d'albumine à 0,1 mg/ml en tampon PBS contenant 0,5% de Tween 20® (PBS/Tween), afin d'éviter l'adsorption de molécules indésirables.

### Exemple 2- Synthèse des particules magnétiques (support libre)

Cette synthèse est décrite dans le document US-C-4,452,773 (Molday), à quelques modifications près.

14 grammes de polysaccharide Dextran® T40 (Mw=40 000, Pharmacia) sont ajoutés à 14 ml d'eau, et on laisse le Dextran se dissoudre à température ambiante (solution 1). Une solution 2 est préparée avec 3 grammes de FeCl₃, 6H₂O (Mw=270,3) et 1,3 grammes de FeCl₂, 4H₂O (Mw=198,81) dans 20 ml d'eau. Les deux solutions sont introduites dans un réacteur à double enveloppe de 250 ml, équipé d'un moteur d'agitation réglé à 200-250 tours/min, d'un agitateur en verre et d'une ampoule de coulée contenant une solution de NH₄OH à 7,5 % (v/v). A température ambiante, la solution de NH₄OH est ajoutée goutte à goutte sous agitation, jusqu'à un pH final compris entre 10 et 11. La température est amenée à 70°C pendant environ 60 minutes, puis la solution finale est dialysée extensivement contre 5 litres d'eau distillée, avec renouvellement des bains de dialyse jusqu'à obtention d'un pH neutre. La solution est ensuite filtrée sur laine de quartz pour éliminer les plus gros agrégats, puis centrifugée 3 fois à 600 tours/min pendant 5 minutes. Afin d'éliminer de Dextran excédentaire, les particules sont déposées sur une colonne de (33 x 2,5) cm de gel de Sephacryl S300 HR (Pharmacia), préalablement équilibrée en tampon acétate 0,1 M, NaCl 0,15 M, 0,05% NaH₃, pH = 6,5. Les particules superparamagnétiques obtenues, revêtues de Dextran, ont un diamètre extérieur compris entre 20 et 900 nm, et préférentiellement compris entre 30 et 100 nm. Elles sont conservées à + 4°C.

Le dosage des particules est réalisé par mesure de la densité optique à 483 nm. Une droite d'étalonnage reliant la densité optique à la masse volumique de la solution déterminée par extrait sec est préalablement établie. Connaissant la densité des particules, on peut ensuite en déduire leur nombre par unité de volume. Elle est typiquement de 15 mg/ml.

Le dosage du fer est effectué par la méthode de Avol, E. et al (Prep. Bioch. 3/3:279, 1973) utilisant la bathophénanthroline. La densité optique du complexe fer/bathophénanthroline est mesurée à 535 nm. La courbe d'étalonnage est réalisée sur une solution de sel de Fer II de molarité connue. Le pourcentage pondéral de fer dans les particules est d'environ 50 (± 10) %.

Le dosage du Dextran est effectué par la technique décrite par Molday, R.S. et al (FEBS Lett., 170/2:232, 1973). La densité optique des complexes formés entre le polysaccharide et le mélange phénol-acide sulfurique est mesurée à 487 nm. La courbe d'étalonnage est réalisée sur du Dextran libre. Le pourcentage pondéral de Dextran dans les particules est d'environ 50 (± 10) %.

### Exemple 3- Fabrication de conjugués particules/anticorps anti-TSH, pour obtenir le réactif

### 3.1) Couplage direct des anticorps anti-TSH sur les particules de l'exemple 2

Les particules sont dialysées en tampon acétate (CH₃COONa 0,1 M, NaCl 0,15 M, pH = 6,5). 5 µl/mg de particules d'une solution de périodate de sodium à 0,1M dans le tampon acétate sont ajoutées, et le temps de réaction est de 45 minutes à l'abri de la lumière. Les particules ainsi oxydées sont dialysées dans le tampon de couplage (phosphate 0,25M, NaCl 0,75M), et les anticorps anti-TSH sont ajoutés (10 à 100 µg/mg de particules). Le couplage a lieu pendant une nuit à 37°C sous agitation.

La liaison imine est ensuite réduite par addition du borohydrure de sodium (0,1M, 1 µmole/mg de particules) durant 15 à 30 minutes à température ambiante sous agitation. Le mélange est finalement dialyse contre un tampon phosphate 0,1 M, NaCl 0,15 M à pH 7,4.

### 3.2) Utilisation de particules commerciales couplées à la streptavidine

### 3.2.1) Biotinylation des anticorps anti-TSH:

2 mg d'anticorps anti-TSH sont dialysés en tampon PBS, puis mis en contact 1 heure avec la biotine-NHS (Pierce) sous agitation, à température ambiante (20 moles de biotine/mole d'anticorps). Les anticorps biotinylés sont de nouveau dialysés pour éliminer l'excès de biotine.

### 3.2.2) Couplage des anticorps biotinylés sur les particules-streptavidine:

2 µg d'anticorps biotinylés tels qu'obtenus précédemment sont ajoutés à 100 µl d'une solution contenant 10¹¹ particules couplées à la streptavidine. Le mélange est incubé pendant 1 heure à température ambiante. Les anticorps libres sont éliminés par passage de la solution sur une colonne contenant une matrice métallique permettant la séparation par gradient de champ magnétique (HGMS) des conjugués formés. De telles colonnes sont, par exemple, commercialisées par Miltenyi Biotec GmbH (Bergisch Gladbach, Germany). Et les techniques correspondantes d'enrichissement sont décrites par les documents US-C-4,375,407, US-C-5,543,289, WO-A-96/26782, WO-A-96/26011, US-C-5,186,827,. et la publication de B.L Hirschbein et al (Chemtech, Mars 1982, pages 172-179). La colonne est rincée par 2 ml de PBS, puis les particules de conjugués sont éluées dans 100 µl de PBS/Tween. On récupère ainsi environ 70% (7x10¹⁰ particules) des particules initiales, sous forme conjuguée. Une courbe de titration montre qu'un à deux anticorps sont immobilisés par particule.

### Exemple 4- Mise en contact du réactif avec l'analyte, et enrichissement du milieu intermédiaire obtenu

1,6x10¹⁰ particules de conjugués telles qu'obtenues à l'exemple 3 sont diluées en PBS/Tween® dans des volumes variables (de 0,1 à 100 ml). 6 ng de TSH sont ajoutés à chaque solution et les essais sont incubés durant 2 heures à 37°C sous agitation. Chaque solution est ensuite déposée et enrichie sur la colonne décrite dans l'exemple 3, puis lavée par 1 ml de PBS/Tween afin d'éliminer les antigènes libres. Pour récupérer les particules, la colonne est retirée de l'aimant. 30 µl de PBS/Tween sont déposés et éliminés, car leur concentration en particules est trop faible. 70 µl sont ensuite déposés et recueillis.

Chaque échantillon de 70 µl est ensuite déposé sur une plaquette telle qu'obtenue dans l'exemple 1. La solution est laissée à incuber durant 1 heure à température ambiante. Les plaquettes sont ensuite lavées vigoureusement par le PBS/Tween, puis à l'eau.

A titre de comparaison, des essais sont réalisés avec des solutions de volume constant (70 µl) dans lesquelles sont mélangés comme précédemment 1.6x10¹⁰ particules de conjugué (réactif) et des masses variables de TSH, de manière à obtenir une gamme de concentrations comparables à celles décrites ci-dessus (de 0,06 à 86 ng/ml). Les solutions ne sont pas enrichies sur colonne selon la technique HGMS, mais directement déposées sur les plaquettes telles qu'obtenues dans l'exemple 1, et incubées dans les même conditions que celles décrites ci-dessus.

La détection du dépôt, concentré, des particules complexes superparamagnétiques, après réaction spécifique sur le support, est réalisée par microscopie à force atomique ou AFM (Autoprobe, Park Scientific Instrument). Les échantillons sont séchés à l'azote, et analysés en mode contact à l'air (pointes Si₃N₄, épaisseur = 6 µm, constante de raideur de 0,01 à 0,5 N/m). Un scanner de 100 µm est utilisé et des balayages de 5x5 µm² sont réalisés. Le nombre de particules détectées sur chaque zone analysée est calculé par un logiciel de traitement d'image et la valeur trouvée est ramenée à un nombre de particules par µm².

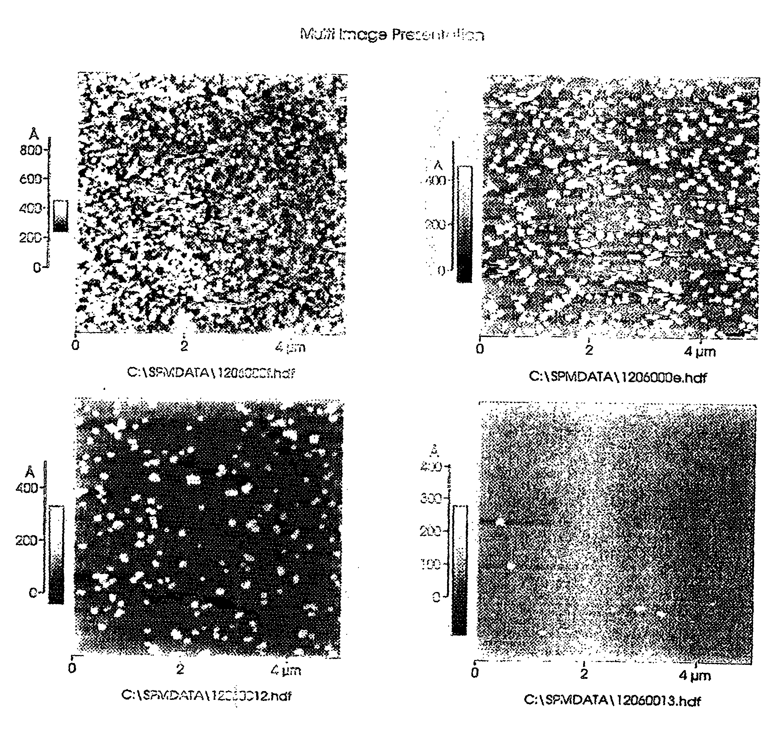

Les résultats sont décrits par la figure 11. Pour des concentrations égales en TSH, une nette augmentation du nombre de particules par unité de surface est observée dans le cas de la concentration selon la technique HGMS. Les limites de détection (calculées comme indiqué à l'exemple 7) pour les deux méthodes sont reportées sur le Tableau 2.

### Exemple 5- Application d'un aimant en relation avec les surfaces limitées, biospécifiques, pendant leur mise en contact avec les particules complexes

4 plaquettes de silice de 8 x 8 mm² sont fonctionnalisées et conjuguées sur toute leur surface par des anticorps anti-TSH de capture, comme décrit dans l'exemple 1. Deux des quatre plaquettes sont placées sur un aimant constitué de terres rares (diamètre 6 mm, hauteur 25 mm, B= 0,32 Tesla), les deux autres sont en dehors de l'action de tout champ magnétique. 70 µl de solutions contenant une concentration en TSH de 1 ou de 100 ng/ml et une concentration identique en particules conjuguées (2x10⁹) sont déposés sur ces plaquettes. Les solutions sont laissées à incuber durant 1 heure à température ambiante sous agitation, afin de limiter l'adsorption non spécifique des particules conjuguées. Les plaquettes sont ensuite analysées par AFM selon le principe décrit dans l'exemple 4, et les résultats sont illustrés par les images de la figure 12. Bien que, dans cette mise en oeuvre particulière, des particules magnétiques de faibles dimensions soient utilisées, les volumes faibles employés permettent malgré tout, leur séparation par un aimant permanent car les distances de séparation sont faibles.

Les deux plaquettes placées sur les aimants montrent une densité en particules nettement supérieure à celles des plaquettes n'ayant pas subi l'influence du champ magnétique. Il a été démontré que le bruit de fond (absence de TSH en solution) est comparable dans les deux cas, et donc que l'amélioration de la réaction anticorps/antigène sur la surface biospécifique est attribuable à la présence de l'aimant. Les résultats sont reportés sur le tableau 1.

**Tableau 1**

| *Concentration en TSH (ng/ml)* | *Nombre de particules / µm*^{*2*} *(sans aimant)* | *Nombre de particules / µm*^{*2*} *(avec aimant)* |
|---|---|---|
| 1 | 0,3 | 8,0 |
| 100 | 33 | 128 |

### Exemple 6- Enrichissement du milieu intermédiaire sur colonne HGMS, puis application d'un aimant en relation avec la surface limitée, biospécifique: Influence de la dimension de la surface biospécifique

Les anticorps de capture ou ligands sont couplés sur la silice activée sur des surfaces de taille croissante. Dans ce but, des volumes variables de 1, 10 et 70 µl de solution d'anticorps sont déposés au centre des plaquettes et couvrent respectivement des surfaces de 1,8, 13 et 56 mm². La plus petite surface (0,5 mm²) est réalisée au moyen d'un cache percé, placé au centre de la plaquette. Les solutions de particules conjuguées (7,6x10⁹) et de TSH (60 pg/ml) sont incubées dans 1 ml, puis purifiées sur colonne HGMS selon le protocole décrit dans l'exemple 4. Les plaquettes telles qu'obtenues à l'exemple 1 sont ensuite placées sur les aimants, et les solutions purifiées sont déposées et laissées à incuber. L'analyse par AFM permet la quantification du nombre de particules par µm².

Les résultats sont décrits par la figure 13. Le nombre de particules complexes immobilisées par unité de surface augmente lorsque la surface biospécifique diminue, et donc, lorsque les particules complexes sont concentrées au centre de la surface sous influence de l'aimant. L'effet de concentration est d'autant plus important que la surface sur laquelle peuvent réagir les particules complexes diminue.

### Exemple 7- Enrichissement du milieu intermédiaire sur colonne HGMS, puis application d'un aimant en relation avec la surface utile limitée, biospécifique: Influence de la quantité d'antigène (analyte) en solution, et évaluation de la limite de sensibilité de la méthode.

Les anticorps de capture ou ligands sont couplés sur la silice activée sur une surface de 64 ou 0,5 mm². Les particules conjuguées (8x10⁹ par essai) et les antigènes (analyte) en concentration variable (de 0 à 500 ng/ml) sont incubés dans 1 ml, puis les particules complexes sont enrichies sur colonne HGMS. Les plaquettes fonctionnalisées, c'est-à-dire couplées à leurs propres récepteurs, sont ensuite placées sur les aimants comme le montre les figures 9bis et 10, et les solutions purifiées sont incubées, puis analysées par AFM.

La figure 15 permet de clairement mettre en évidence une nouvelle fois l'influence de la dimension de la surface utile biospécifique, sur l'effet de concentration ou confinementement par aimantation. On observe en effet à concentration en TSH égale une densité de particules complexes beaucoup plus importante sur la petite surface (0,5 mm²), représentée par des croix, par rapport à la grande surface (64 mm²), pour laquelle la densité est représentée par des ronds.

Pour la concentration sur une surface utile de 0,5 mm², les résultats sont décrits par la figure 14. Le calcul démontre que la valeur du plateau obtenu est conforme à la courbe théorique (figure 7, courbe 6bis) attendue. La limite de détection est calculée comme précédemment (exemple 6). selon la méthode statistique généralement admise: 10 blancs (sans antigène) sont réalisés, dont on calcule la rugosité moyenne (Moy_{R}) et l'écart type (ET_{R}). La limite de détection (LDD) est définie comme étant: LDD = Moy_{R} + 3. ET_{R}. Cette valeur est reportée sur la pente à l'origine de la courbe d'étalonnage. Une limite de détection de 5x10⁻³ pg/ml, soit 1.2x10⁵ molécules de TSH est calculée (Tableau 2).

### Comparaison des résultats :

La figure 16 reprend sur un même graphe, les résultats décrits dans les exemples 4 et 7 et permet de mettre en évidence, l'amélioration de la sensibilité apportée par le montage représenté sur la figure 10. Le tableau 2 ci-dessous permet également de montrer l'amélioration de la limite de sensibilité pour la détection de la TSH, apportée par l'enrichissement magnétique sur colonne par gradient de champs d'une part, et par l'effet conjoint de l'enrichissement magnétique du milieu intermédiaire sur colonne par gradient de champ et de la concentration magnétique des particules complexes sur une surface biospécifique de faibles dimensions, d'autre part.

**Tableau 2**

| **Méthode** | **Limite de détection (pg/ml)** | **Surface utile (mm**^{**2**}**)** |
|---|---|---|
| Séparation sur colonne par HGMS | 100 | 64 |
| Séparation sur colonne HGMS et concentration par aimantation | 4 | 64 |
| Séparation sur colonne HGMS et concentration par aimantation sur une surface de faible dimension | 0,005 | 0,5 |

A titre de comparaison, la limite de détection obtenue sur les automates d'immuno-analyse est de l'ordre de quelques pg/ml selon les cas. Un gain en sensibilité d'un facteur d'environ 100 à 1000 est donc obtenu en mettant en oeuvre la méthode selon la présente invention.

### Exemple 8- Synthèse des particules conjuguées d'or-anticorps anti-TSH

L'exemple ci-dessous montre que la méthode selon l'invention, peut être mise en oeuvre et étendue aux particules de support non magnétiques et de nature différente.
- *Solution d'or colloïdal.* Le pH de la solution d'or colloidal (diamètre des particules : 15 nm, Polyscience, DO₅₂₀=0,524), est ajusté à 7,2 par une solution de K₂CO₃ 0,2M. L'or colloïdal est ensuite filtré sur membrane de 0,1 µm.
- *Solution de ligand.* Les anticorps anti-TSH sont dialysés en tampon borate 2 mM pH 9,00, puis filtrés sur membrane de 0,1 µm, juste avant usage, afin d'éliminer les agrégats éventuels, et dosés à 280 nm.
- *Synthèse des particules conjuguées protéines/or*.

La solution d'anticorps est ajoutée à la solution d'or, sous agitation, à une concentration de 8 µg d'anticorps/ml d'or colloïdal. Après 10 minutes d'incubation, la stabilisation colloïdale est assurée par addition d'une solution de sérum-albumine bovine (BSA) à 10 mg/ml en PBS, pour obtenir une concentration finale en particules conjuguées de 100 µg/ml. La solution est filtrée (0,1 µm), puis purifiée sur une membrane de seuil de coupure MWCO= 300,000, afin d'éliminer l'excès d'anticorps. Les particules conjuguées sont reprises en milieu PBS/BSA/Tween 20 0,5%, et filtrées. Elles sont stockées à 4°C en présence d'azoture de sodium (0,05%) et peuvent être conservées pendant plusieurs mois. L'azoture de sodium peut éventuellement être remplacé par une filtration stérilisante au moment de l'emploi.

### Exemple 9- Dosage sandwich de la TSH par les particules conjuguées marquées à l'or colloïdal: Comparaison des méthodes avec et sans enrichissement par centrifugation du milieu intermédiaire

### * Sans enrichissement.

Sur des plaquettes telles qu'obtenues dans l'exemple 1, 70 µl de solution de TSH de concentration croissante de 0 à 50 ng/ml de PBS/Tween 0,5% sont déposés sur les plaquettes et incubés une heure à 37°C. Après rinçage par le PBS/Tween, on incube 70 µl de solution de particules conjuguées (DO 520 nm=1,4) 1h à 37°C. Les plaquettes sont ensuite lavées, séchées, et analysées par AFM. On dénombre le nombre de particules complexes par unité de surface.

### * Avec enrichissement par centrifugation

Après synthèse des particules conjuguées (réactif) précédemment décrites, on incube dans des volumes variables de 0,1 à 10 ml de PBS/Tween 0,5% contenant 0,1 mg/ml de BSA, 100 µl de particules conjuguées (DO-₅₂₀₋ₙₘ=1,4), et 6 ng de TSH. Les solutions obtenues sont incubées une nuit à 37°C sous agitation. Des témoins sans antigène (analyte) sont réalisés. A l'issue de l'incubation, les solutions sont enrichies par lavage et filtration sur membrane (MWCO=100 000), pour obtenir un volume final de quelques dizaines de µl. Elles sont ensuite déposées sur des plaquette telles qu'obtenues dans l'exemple 1. L'incubation a lieu 1h à 37°C. Le rinçage et l'analyse ont lieu comme précédemment.

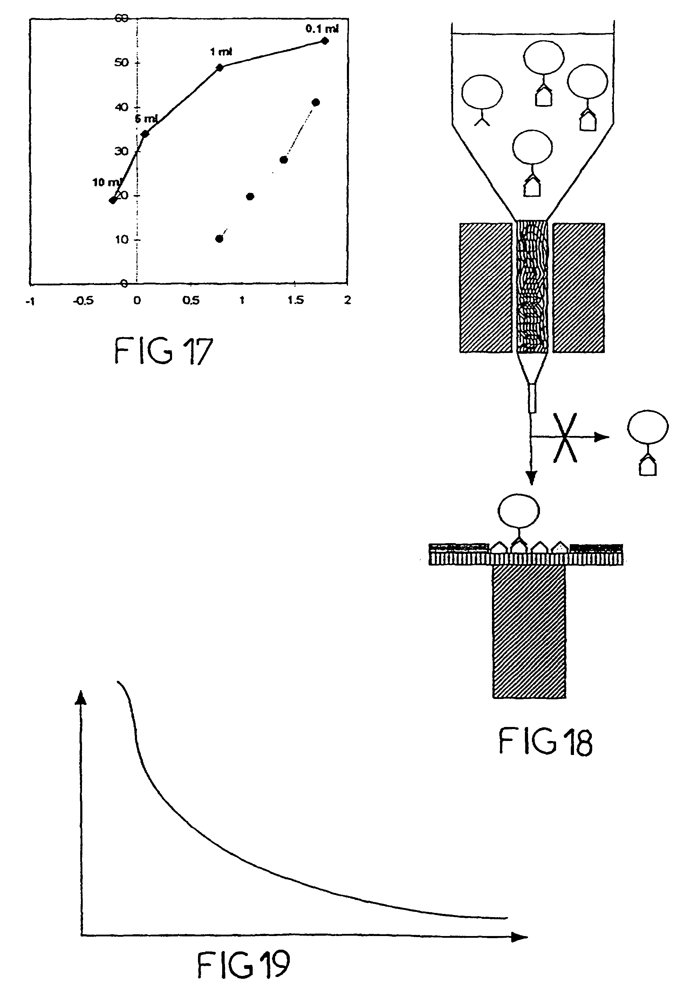

Les résultats sont présentés sur la figure 17. On constate une nette amélioration de la sensibilité à concentration équivalente en TSH, lorsque les particules complexes sont concentrées.

### Exemple 10- Fabrication des supports biospécifiques (autres supports et récepteurs) par couplage d'oligonucléotides (ODN)

Les surfaces de silice sont nettoyées par l'acide sulfochromique (2h, 120°C) puis silanisées par l'aminopropyldimethyléthoxysilane (2% dans le toluène anhydre) pendant 2h à température ambiante. Un copolymère, l'anhydride maléique-co-methyl vinyl éther (AMVE, Mn = 20,000), capable d'établir des liens covalents avec des fonctions amines, est ensuite greffé sur les surfaces aminées. Une solution à 1 mg d'AMVE/10 ml de DMSO anhydre est préparée juste avant usage. Les surfaces aminées sont immergées dans cette solution et incubées sous agitation pendant 1h à température ambiante en présence de triéthylamine (1% v/v). Elles sont ensuite rincées par le DMSO et séchées à l'azote.

Une solution d'ODN de capture (17 bases) est préparée à une concentration de 8 µM dans un mélange borate de sodium 0.1 M, NaCl 0.5M (5%)/DMSO (95%). 50 µl de solution sont déposés sur la plaque et incubés 1 h à 37°C. Les supports sont ensuite rincés en PBS-Tween. Puis 50 µl d'une solution de PEG sont déposés sur la surface et incubés 30 minutes pour éviter les interactions non spécifiques. Les surfaces sont rincées en PBS/Tween. De telles surfaces sont appelées ci-après « surfaces biospécifiques ».

### Exemple 11. Fabrication des conjugués de détection particules magnétiques/ODN-biotine

### Exemple 11a

100 µl de particules magnétiques recouvertes par la streptavidine (Immunicon) sont ajoutés à 100 µl d'une solution de l'ODN de détection (21 bases) biotinylé (8.37 pmole/µl). Cette solution est incubée pendant 1 h à 37°C sous agitation. L'excès d'ODN est éliminé par 3 étapes successives d'aimantation/dilution et les conjugués sont finalement repris dans 100 µl de tampon PEG.

### Exemple 11b.

100 µl de particules magnétiques recouvertes par la streptavidine (10¹¹ particules, soit 5.10¹² molécules de streptavidine, Miltenyi Biotec) sont ajoutées à la solution de l'ODN (28 bases) de détection biotinylé (4.10¹³ molécules), porteur d'un brin espaceur de huit bases. Cette solution est incubée 1 h à 37°C sous agitation. L'excès d'ODN est éliminé par passage sur colonne de séparation HGMS puis repris dans 100 µl.

Les deux réactifs ainsi obtenus sont appelés ci-après "conjugués de détection"

### Exemple 12. Capture des cibles ADN (analyte) par les conjugués de détection de l'exemple 11a.

20 µl de la solution de conjugués de détection tels que décrit dans l'exemple 11a sont dilués dans le tampon PEG. Les cibles (37 bases, 8.23. 10¹³ copies/µl), portant les séquences respectivement complémentaires des ODN de capture et de détection, sont ajoutées en quantité désirée pour un volume total de 1 ml. Le mélange est incubé durant 2 h à 37°C sous agitation. Les complexes formés seront utilisés comme tels.

### Exemple 13. Concentration de cibles (analyte) à l'aide des conjugués de détection de l'exemple 11a. Influence de la présence de l'aimant.

2 supports biospécifiques sont préparés. Egalement, 2 échantillons contenant chacun 10¹² copies/ml sont incubés en présence de conjugués de détection tels que décrits dans l'exemple 11a. Les supports biospécifiques sont placés au fond d'une cuve. L'une de ces cuves est placée sur un aimant constitué de terres rares (diamètre 6 mm, hauteur 6 mm, B=0,32 Tesla). La solution contenant les cibles hybridées sur les particules magnétiques est déposée dans les cuves, et incubée durant 1 h sous agitation par pipetage toutes les 10 min. A l'issue de l'incubation, les supports sont rincés en PBS-Tween puis en tampon carbonate d'ammonium 0. 1 M, avant d'être séchés et analysés en mode contact à l'air par AFM.

Dans une zone utile de 4 x 4 µm de la surface biospécifique, 0,3 particule par µm² sont détectées sans aimant, et 86 particules par µm² avec un aimant.

Les cibles ne sont pratiquement pas détectées en l'absence de l'aimant, alors qu'une monocouche de particules est obtenue en présence de l'aimant, correspondant à la saturation du signal.

### Exemple 14. Concentration de cibles (analyte) à l'aide de conjugués de détection de l'exemple 11a. Détermination de la limite de détection.

Plusieurs supports biospécifiques sont préparés. Des concentrations croissantes en cibles (entre 0 et 8.10¹² cibles/ml) sont préparées dans 1 ml de tampon PEG. La capture des cibles ADN par les conjugués de détection tels que décrits dans l'exemple 11a est réalisée comme décrit dans l'exemple 12. A l'issue de l'incubation, les supports biospécifiques sont placés sur des aimants tels que décrits à l'exemple 12, à une température d'incubation de 37°C. 100 µl des solutions contenant les cibles hybridées sur les conjugués de détection sont déposés sur les supports biospécifiques. La solution est incubée durant 3 minutes puis le surnageant est éliminé. 100 µl sont de nouveau déposés, puis la goutte est agitée par pipetage afin de remettre les particules en suspension. Ce protocole est répété jusqu'à ce que toute la solution ait été concentrée sur le support (10x100 µl). A l'issue de l'incubation, les surfaces sont rincées par le PBS-Tween puis par tampon carbonate d'ammonium 0.1 M, avant d'être séchées et analysées en mode contact à l'air par AFM. La densité des particules est mesurée dans la zone d'aimantation maximale. Le taux de recouvrement des particules sur les images est déterminé par l'utilisation d'un logiciel de traitement d'images.

La limite de sensibilité est estimée à 7.10⁸ cibles/ml, soit un gain d'un facteur 100 par rapport à la détection de cibles ADN par un test enzymatique conventionnel, type ELOSA (Enzym Linked Oligo Sorbent Assay), sans concentration (8.10¹⁰ cibles/ml), réalisé sur le même support.

### Exemple 15. Concentration de cibles (analyte) à l'aide des conjugués de détection de l'exemple 11b. Détermination de la limite de détection.

50 µl de la solution de conjugués de détection tels que décrits dans l'exemple 11b sont dilués en tampon PEG. Les cibles (37 bases), portant les séquences respectivement complémentaires des ODN de capture et de détection, sont ajoutées en quantité désirée pour un volume total de 1 ml. Le mélange est incubé durant la nuit à 37°C sous agitation. Les complexes conjugués de détection-cibles sont ensuite purifiés sur colonne de séparation HGMS telle que décrite à l'exemple 3, pour obtenir un volume final de 100 µl.

Plusieurs supports biospécifiques sont préparés. Ils sont placés sur des aimants tels que décrits à l'exemple 13, à une température d'incubation de 37°C. La solution de complexes conjugués de détection-cibles est incubée durant 30 minutes avec une agitation par pipetage toutes les 10 minutes. A l'issue de l'incubation, les surfaces sont rincées par le PBS-Tween puis en tampon carbonate d'ammonium 0.1 M, avant d'être séchées et analysées en mode contact à l'air par AFM.

La limite de détection est estimée à 3.10⁹ copies/ml, soit un gain d'un facteur 30 par rapport au test ELOSA réalisé sur le même support sans concentration.

### Exemple 16. Comparaison du système de concentration en solution, par rapport à une incubation séquencée des réactifs en surface

**Essai 1:** les cibles sont capturées en solution comme décrit à l'exemple 15.

**Essai 2:** après préparation des supports biospécifiques, 100 µl d'une solution de cibles (entre 0 et 8.10¹³ cibles/ml) sont incubés sur le support (particules) qui est ensuite rincé par le PBS-Tween. Puis 100 µl de solution d'ODN biotinylés (0.5 µM en PEG) sont incubés. Enfin, le support est placé sur un aimant cylindrique tel que décrit à l'exemple 13, et 100 µl d'une solution commerciale de particules magnétiques recouvertes de streptavidine (Miltenyi Biotec) et diluées au demi par le tampon PEG sont incubés durant 30 minutes avec une agitation toutes les 10 minutes.

La limite de détection du système séquencé en surface est estimée à 7.6.10¹⁰ cibles/ml, alors que celle de l'essai en solution avait été estimée à l'exemple 15 à 3.10⁹ copies/ml. Le facteur d'amplification lié à l'incubation des cibles et des conjugués en solution est donc de 25.

### Exemple 17. Intérêt de la cellule à circulation dans le but de concentrer des conjugués magnétiques

Cet essai illustre un autre procédé de mise en oeuvre de l'invention représenté aux figures 23 et 24.

Des supports biospécifiques sont préparés en adsorbant des anticorps antiferritine biotinylés (témoin positif) ou non biotinylés (témoin négatif) à une concentration de 0.2 µl/ml sur des surfaces de silice.

**Essai 1:** Ces supports sont placés dans la cellule contenant un aimant cylindrique tel que défini précédemment, surmonté d'un cône tronqué de 0.5 mm de diamètre à son sommet. Une solution contenant 30 µl de particules magnétiques recouvertes de streptavidine (Miltenyi Biotec), diluée dans 1 ml de tampon PBS/Tween/BSA (0. 1 mg/ml), est injectée sur chaque support à un débit de 0.017 ml/min. A l'issue de cette incubation, les supports sont rincés, séchés et analysés en AFM.

**Essai 2:** Parallèlement, des essais sont réalisés sur ces mêmes supports biologiques en dehors de la cellule. Chaque support est placé sur un aimant cylindrique tel que défini précédemment. Une solution contenant 30 µl de particules magnétiques recouvertes de streptavidine (Miltenyi Biotec), diluée dans 60 µl de tampon PBS-Tween-BSA est préparée et déposée sur les supports, puis incubée durant 30 minutes en agitant toutes les 10 minutes. A l'issue de cette incubation, les supports sont rincés, séchés et analysés en AFM et le taux de recouvrement des particules est évalué.

On constate i) que le signal spécifique (97+/-3 pour l'essai 1, 63+/-16) pour l'essai 2 et ii) et le rapport signal/bruit (139 pour l'essai 1, 31 pour l'essai 2) sont nettement améliorés lors de l'utilisation de la cellule à circulation.

En résumé, les exemples décrits ci-dessus matérialisent le procédé général selon l'invention. L'exemple 7 montre que l'invention permet de détecter des concentrations en analyte aussi faibles que 5 fg/ml de TSH, ce qui correspond à 1.2x10⁵ molécules. Une bactérie représente environ 10⁴ à 10⁵ molécules d'ARN ribosomique. Les exemples 10 à 16 montrent que des procédés de mise en oeuvre, identiques ou différents, peuvent être utilisés pour la détection de cibles ADN. Par conséquent, avec une mise en oeuvre du même type, il apparaît possible de détecter de 1 à 10 bactéries par unité de volume. Associée à une méthode de concentration telle que décrite et appliquée à du matériel nucléique, la sensibilité analytique de la méthode selon l'invention est donc du même ordre que celle de méthodes d'amplification comme PCR, NASBA, ...

Les modes de mise en oeuvre de l'invention peuvent être multiples et, sans que cela soit limitatif, on peut en donner quelques exemples:

L'invention peut être appliquée aux dosages d'haptènes par compétition selon un exemple de procédé décrit sur la figure 18. Sur une surface limitée, des haptènes sont immobilisés. La solution contenant des haptènes à doser (analyte) est placée au contact de particules conjuguées ne portant qu'un anticorps anti-haptène (récepteur) ainsi que décrit à l'exemple 3, pendant un temps suffisant pour que les particules complexes se forment. Les particules complexes sont séparées selon l'un des procédés décrits et concentrées sur la surface utile portant les haptènes récepteurs. Les particules conjuguées ayant préalablement réagi avec un haptène en solution, ne peuvent se fixer et sont éliminées par lavage. Les particules conjuguées n'ayant pas réagi, porteuses de l'anticorps disponible peuvent, au contraire, réagir avec les haptènes récepteurs de la surface utile, biospécifique et être détectées par l'un des différents moyens décrits précédemment. Le nombre de particules conjuguées détectées diminuent quand la concentration en haptènes en solution augmente, comme cela est représenté sur la courbe théorique de la figure 19.

Tel que décrit dans les documents US-C-5,543,289 ou US-C-5,169,754, des cellules peuvent être séparées d'une solution par un tri magnétique. A la suite de cette opération, il est possible de concentrer l'analyte marqué (cellules, levures, bactéries, virus ou phages) et de le retenir sur une surface biospécifique de faible dimension, par un récepteur différent de ou identique à celui qui a permis l'obtention des particules complexes, par un procédé tel que décrit sur la figure 10 par exemple. La détermination (détection et/ou quantification) peut être faite par tous moyens appropriés, tels que des marqueurs fluorescents intrinsèques ou extrinsèques, méthodes de microscopies, ... y compris la détection par l'utilisation des particules ayant servi à la séparation et à la concentration.

Des mises en oeuvre particulières, visant notamment à lier plusieurs ligands possédant des affinités différentes sur une même surface limitée (multiaffinité), et/ou à concentrer les particules conjuguées en des emplacements désirés permet d'appliquer l'invention à une détection multiple. Un exemple est décrit sur la figure 20; dans ce cas, la surface utile comprend trois zones utiles A, B et C comprenant trois récepteurs respectivement différents, et situées à trois positions géographiques identifiées. Par un procédé de mise en oeuvre particulier, tel que décrit sur la figure 21, on immobilise des particules conjuguées sur leurs récepteurs spécifiques. Le procédé comprend un dispositif 14 qui comprend un compartiment 15 dans lequel a lieu la formation des particules complexes 5. Ce compartiment peut comprendre un système d'agitation mécanique, ou, si les particules utilisées sont magnétiques, un système d'agitation par champ magnétique. Après un temps suffisant pour la formation des particules complexes, celles-ci sont amenées au contact de la surface 17 (mono ou multiaffinité) par l'intermédiaire d'un canal 16; ce dernier, de hauteur faible, vise à limiter la hauteur de solution au dessus de ladite surface, limitant ainsi la distance qu'une particule complexe doit parcourir avant d'être capturée par son récepteur spécifique. La 'probabilité de réaction particule complexe/récepteur peut être avantageusement améliorée par recirculation de la solution sur la surface limitée. Le dispositif décrit l'utilisation d'un aimant 12 qui se déplace d'une position vers la suivante (par exemple, de la zone A vers la zone B, puis vers la zone C). Un aimant unique occupe selon une autre variante la totalité de la surface, ou plusieurs aimants, chacun en regard d'une zone A, B ou C, sans que cela change le procédé de l'invention. Le dispositif décrit peut par conséquent être utilisé pour la multidétection d'analytes, ou pour le criblage de banques (de phages, par exemple).

Les dispositifs décrits précédemment peuvent être réalisés selon des techniques issues des microtechnologies.

## Revendications

1. Procédé pour isoler au moins un analyte (4) dans un milieu, dans lequel il est distribué, procédé selon lequel:
(a) on dispose d'au moins un réactif (3) comprenant un support à l'état libre, sous forme discrète, exhibant au moins un récepteur de l'analyte
(b) on dispose d'un autre support, de capture, dont la surface accessible comprend au moins une zone utile ou active, de surface limitée, exhibant au moins un autre récepteur (10) d'une entité à capturer, à savoir dudit analyte ou dudit récepteur,
**caractérisé en ce que**
(c) on met en contact le réactif (3) avec un échantillon liquide obtenu à partir du milieu contenant l'analyte, pour former un milieu intermédiaire comprenant, toujours sous forme discrète et distribuée dans ledit milieu, un complexe entre ledit support, le récepteur et l'analyte,
(d) on met en contact la surface accessible dudit autre support avec au moins un partenaire de capture, à savoir le réactif n'ayant pas réagi lors de l'étape (c) ou le complexe, pour lier ledit partenaire de capture à ladite surface accessible, et obtenir un dépôt concentré dans ladite zone utile, immobilisé sur ledit autre support par la liaison dudit autre récepteur avec ledit récepteur ou l'analyte,
(e) on concentre le dépôt dudit partenaire de capture dans ladite zone utile, en augmentant la quantité dudit partenaire par unité de surface dans ladite zone utile.

2. Procédé selon la revendication 1, **caractérisé en ce que**, selon l'étape (e), le taux de concentration en partenaire de capture est déterminé pour que la quantité dudit partenaire de capture immobilisé demeure au plus égale à la quantité dudit autre récepteur sur ledit autre support.

3. Procédé selon la revendication 2, selon lequel le réactif comprend des particules du support conjugué avec le récepteur, ayant la capacité d'être séparées de tout milieu liquide dans lequel elles sont dispersées, sous l'action d'un moyen physique appliqué au milieu liquide, **caractérisé en ce qu'**on applique ledit moyen physique à au moins une partie du milieu intermédiaire, pour séparer lesdites particules complexes, éventuellement on lave lesdites particules complexes ainsi séparées, et on élue lesdites particules complexes, en cessant d'appliquer ledit moyen physique, pour concentrer en partenaire de capture ledit dépôt dans ladite zone utile.

4. Procédé selon la revendication 3, **caractérisé en ce que** le réactif comprend des particules du support, magnétiques, en particulier superparamagnétiques, et le moyen physique consiste en un champ magnétique appliqué au milieu intermédiaire, par exemple selon la technique dite HGMS (High Gradient Magnetic Separation).

5. Procédé selon la revendication 1, **caractérisé en ce que** ledit récepteur et ledit autre récepteur comportent le même ligand, immobilisé respectivement sur ledit support et ledit autre support.

6. Procédé selon la revendication 1, selon lequel le réactif comprend des particules du support conjugué avec le récepteur, ayant la capacité d'être séparées de tout milieu liquide dans lequel elles sont dispersées, sous l'action d'un moyen physique appliqué audit milieu liquide, **caractérisé en ce qu'**on applique ledit moyen physique en relation avec ledit autre support, pour concentrer ledit dépôt dans ladite zone utile.

7. Procédé selon la revendication 1, **caractérisé en ce que** le réactif est marqué, et le traitement de l'analyte comprend une étape de détermination du marqueur du dépôt concentré, immobilisé dans ladite zone utile dudit autre support.

8. Procédé selon la revendication 7, **caractérisé en ce que** le marquage du réactif est obtenu selon au moins l'un des modes suivants, à savoir:
- le support constitue lui-même un marqueur
- un marqueur est lié audit support
- un marqueur est lié audit récepteur.

9. Procédé selon la revendication 7, **caractérisé en ce qu'**on détermine l'analyte à partir du dépôt concentré dans ladite zone utile, par une méthode choisie dans le groupe incluant les méthodes topographiques, par exemple par microscopie à force atomique (en abrégé AFM), les méthodes magnétiques, par exemple par microscopie à force magnétique, les méthodes électriques, par exemple mesure d'une variation de capacité ou de résistance, les méthodes optiques, par exemple mesure de l'indice de réfraction, et les méthodes de mesure de variations de masse, par exemple microbalance à cristal de quartz.

10. Procédé selon la revendication 9, **caractérisé en ce que** la zone utile est dimensionnée pour que la quantité du dépôt immobilisé sur ledit autre support soit au moins égale au seuil de sensibilité de la méthode de détermination, exprimé en nombre de particules par unité de surface.

11. Procédé selon la revendication 1, selon lequel le format d'essai est direct, **caractérisé en ce que** le partenaire de capture est ledit complexe entre ledit support, le récepteur et l'analyte.

12. Procédé selon la revendication 1, selon lequel le format d'essai est par compétition, **caractérisé en ce que** le partenaire de capture est ledit réactif.

13. Procédé selon la revendication 1, **caractérisé en ce que**, à partir du milieu intermédiaire, on obtient un autre échantillon, en enrichissant ledit milieu intermédiaire en complexe, et ledit autre échantillon est mis en contact avec la surface accessible dudit autre support.

14. Procédé selon la revendication 13, **caractérisé en ce qu'**on met en contact ledit autre échantillon avec la surface accessible dudit autre support, en déposant une quantité dosée dudit autre échantillon sur la surface accessible de ce dernier.

15. Procédé selon la revendication 13, **caractérisé en ce qu'**on met en contact ledit autre échantillon avec ladite surface accessible dudit autre support, par passage d'un courant dudit autre échantillon, d'épaisseur relativement faible, éventuellement recyclé, au contact de ladite surface accessible.

16. Procédé selon la revendication 1, selon lequel le milieu liquide contient une pluralité d'analytes différents, **caractérisé en ce que** la surface accessible dudit autre support comprend une pluralité de zones utiles, exhibant une pluralité d'autres récepteurs respectivement différents, de partenaires de capture respectivement différents.

## Patentansprüche

1. Verfahren zum Isolieren von zumindest einem Analyten (4) in einem Medium, in welchem dieser verteilt ist, wobei das Verfahren folgende Schritte aufweist, nämlich:
a) Bereitstellen zumindest eines Reagenz (3), welches einen Träger in einem freien Zustand und von diskreter Form aufweist, und welches zumindest einen Rezeptor für den Analyten enthält,
b) Bereitstellen zumindest eines weiteren Trägers, nämlich zum Einfangen, dessen zugängliche Oberfläche zumindest einen geeigneten oder aktiven Bereich mit einer begrenzten Oberfläche aufweist, wobei dieser zumindest einen weiteren Rezeptor (10) für ein einzufangendes Gebilde, nämlich den Analyten oder den Rezeptor, aufweist,
**gekennzeichnet durch**,
c) in Kontakt bringen des Reagenz (3) mit einer flüssigen Probe, welche aus dem den Analyten enthaltenden Medium gewonnen wird, zur Bildung eines Zwischenmediums, das, nach wie vor in diskreter Form und in dem Medium verteilt einen Komplex zwischen dem Träger, dem Rezeptor und dem Analyten enthält,
d) in Kontakt bringen der zugänglichen Oberfläche des weiteren Trägers mit zumindest einem zu fangenden Partner, nämlich dem Reagenz, das beim Schritt (c) nicht reagiert hat oder dem Komplex, um den zu fangenden Partner an die zugängliche Oberfläche zu binden, und um eine konzentrierte Ablagerung in dem geeigneten Bereich zu erzielen, wobei diese auf dem weiteren Träger **durch** die Bindung zwischen dem weiteren Rezeptor mit dem Rezeptor und dem Analyten immobilisiert ist,
e) Konzentrieren der Ablagerung des zu fangenden Partners in dem geeigneten Bereich, um die Menge des genannten Partners pro Flächeneinheit in dem geeigneten Bereich zu erhöhen.

2. Verfahren nach Anspruch 1, **dadurch gekennzeichnet, daß** in Schritt e) der Grad der Aufkonzentrierung an zu fangendem Partner so bestimmt wird, daß die Menge des genannten immobilisierten zu fangenden Partners höchstens gleich der Menge des weiteren Rezeptors auf dem weiteren Träger wird.

3. Verfahren nach Anspruch 2, bei dem das Reagenz Trägerpartikel aufweist, die mit dem Rezeptor konjugiert sind, wobei diese die Eigenschaft haben, daß sie von jeglichem flüssigen Medium in dem diese dispergiert sind unter der Wirkung eines physikalischen Mittels, das auf das flüssige Medium einwirkt getrennt zu werden, **dadurch gekennzeichnet, daß** man das physikalische Mittel auf zumindest einen Teil des Zwischenmediums einwirken läßt, um die Partikelkomplexe abzutrennen, wobei ggf. die so abgetrennten Partikelkomplexe gewaschen werden, und daß man die Partikelkomplexe eluiert, wobei die Einwirkung des physikalischen Mittels eingestellt wird, um die Ablagerung in dem geeigneten Bereich an einzufangendem Partner zu konzentrieren.

4. Verfahren nach Anspruch 3, **dadurch gekennzeichnet, daß** das Reagenz magnetische, insbesondere superparamagnetische Trägerpartikel enthält, und daß das physikalische Mittel aus einem Magnetfeld besteht, das auf das Zwischenmedium einwirkt, beispiels mittels einer HGMS (High Gradient Magnetic Separation) genannten Technik.

5. Verfahren nach Anspruch 1, **dadurch gekennzeichnet, daß** der Rezeptor und der weitere Rezeptor den gleichen Liganden aufweisen, welcher jeweils auf dem Träger und dem weiteren Träger immobilisiert ist.

6. Verfahren nach Anspruch 1, bei dem das Reagenz Trägerpartikel aufweist, die mit dem Rezeptor konjugiert sind, wobei diese die Eigenschaft aufweisen, daß sie von jeglichem flüssigem Medium in dem diese dispergiert sind unter der Wirkung eines physikalischen Mittels, das auf das flüssige Medium einwirkt getrennt zu werden, **dadurch gekennzeichnet, daß** man das physikalische Mittel in Zusammenhang mit dem weiteren Träger anwendet, um die Ablagerung in dem geeigneten Bereich zu konzentrieren.

7. Verfahren nach Anspruch 1, **dadurch gekennzeichnet, daß** das Reagenz markiert ist, und daß die Behandlung des Analyten einen Schritt zur Bestimmung der Markierung der konzentrierten Ablagerung aufweist, die im geeigneten Bereich des anderen Trägers immobilisiert ist.

8. Verfahren nach Anspruch 7, **dadurch gekennzeichnet, daß** die Markierung des Reagenz auf zumindest einer der folgenden Art und Weisen erhalten wird, nämlich:
- der Träger selbst bildet einen Marker
- ein Marker ist mit dem Träger verbunden
- ein Marker ist mit dem Rezeptor verbunden.

9. Verfahren nach Anspruch 7, **dadurch gekennzeichnet, daß** der Analyt aus der konzentrierten Ablagerung in dem geeigneten Bereich bestimmt wird, und zwar durch ein Verfahren ausgewählt aus der Gruppe die folgendes einschließt, nämlich topographische Methoden, bspw. durch Atomkraftmikroskopie (abgekürzt AFM), magnetische Verfahren, z. B. durch Magnetkraftmikroskopie, elektrische Methoden, bspw. durch Messung einer Änderung der Kapazität oder des Widerstandes, optische Methoden, bspw. durch Messen des Refraktionindexes, und Verfahren zur Messung von Massenänderungen, bspw. durch eine Mikroanalysenwaage mit einem Kristallquarz.

10. Verfahren nach Anspruch 9, **dadurch gekennzeichnet, daß** der geeignete Bereich so dimensioniert ist, daß die Menge der immobilisierten Ablagerung auf dem weiteren Träger zumindest gleich der Sensibilisierungsschwelle des Bestimmungsverfahrens ist ausgedrückt in der Anzahl der Partikel pro Oberflächeneinheit.

11. Verfahren nach Anspruch 1, bei dem die Art des Assays direkt ist, **dadurch gekennzeichnet, daß** der zu fangende Partner der Komplex zwischen dem Träger, dem Rezeptor und dem Analyten ist.

12. Verfahren nach Anspruch 1, bei dem die Art des Assays konkurrierend ist, **dadurch gekennzeichnet, daß** der zu fangende Partner das Reagenz ist.

13. Verfahren nach Anspruch 1, **dadurch gekennzeichnet, daß** aus dem Zwischenmedium eine weitere Probe gewonnen wird, in dem das Zwischenmedium am Komplex angereichert wird, und die weitere Probe wird mit der zugänglichen Oberfläche des weiteren Trägers in Kontakt gebracht.

14. Verfahren nach Anspruch 13, **dadurch gekennzeichnet, daß** die weitere Probe mit der zugänglichen Oberfläche des weiteren Trägers in Kontakt gebracht wird, indem eine dosierte Menge der weiteren Probe auf die zugängliche Oberfläche des letzteren gebracht wird.

15. Verfahren nach Anspruch 13, **dadurch gekennzeichnet, daß** die weitere Probe mit der zugänglichen Oberfläche des weiteren Trägers in Kontakt gebracht wird, indem man einen Strom der weiteren Probe mit einer relativ geringen Dicke, ggf. wiederholt, in Berührung mit der zugänglichen Oberfläche strömen läßt.

16. Verfahren nach Anspruch 1, bei dem das flüssige Medium eine Vielzahl an unterschiedlichen Analyten aufweist, **dadurch gekennzeichnet, daß** die zugängliche Oberfläche des weiteren Trägers eine Vielzahl an geeigneten Bereichen aufweist, die eine Vielzahl an jeweils unterschiedlichen weiteren Rezeptoren für entsprechend unterschiedliche zu fangende Partner aufweisen.

## Claims

1. Method for isolating at least one analyte (4) in a medium, in which it is distributed, method according to which:
(a) at least one reagent (3) is available comprising a support in the free state, in discrete form, exhibiting at least one receptor for the analyte
(b) another support, for capture, is available whose accessible surface comprises at least one useful or active zone, of limited surface, exhibiting at least one other receptor (10) for an entity to be captured, namely for the said analyte or for the said receptor,
**characterized in that**
(c) the reagent (3) is brought into contact with a liquid sample obtained from the medium containing the analyte, to form an intermediate medium comprising, still in a discrete form and distributed in the said medium, a complex between the said support, the receptor and the analyte,
(d) the accessible surface of the said other support is brought into contact with at least one capture partner, namely the reagent which has not reacted during step (c), or the complex, to bind the said capture partner to the said accessible surface, and to obtain a deposit concentrated in the said useful zone, immobilized on the said other support by the binding of the said other receptor with the said receptor or the analyte,
(e) the deposit of the said capture partner is concentrated in the said useful zone by increasing the quantity of the said partner per unit of surface in the said useful zone.

2. Method according to Claim 1, **characterized in that**, according to step (e), the capture partner concentration level is determined so that the quantity of the said immobilized capture partner remains at most equal to the quantity of the said other receptor on the said other support.

3. Method according to Claim 2, according to which the reagent comprises particles of the support conjugated with the receptor, having the capacity to be separated from any liquid medium in which they are dispersed, under the action of a physical means applied to the liquid medium, **characterized in that** the said physical means is applied to at least part of the intermediate medium, in order to separate the said complex particles, the said complex particles thus separated are optionally washed, and the said complex particles are eluted, by ending the application of the said physical means, in order to concentrate in relation to the capture partner the said deposit in the said useful zone.

4. Method according to Claim 3, **characterized in that** the reagent comprises particles of the support, which are magnetic, in particular superparamagnetic, and the physical means consists of a magnetic field applied to the intermediate medium, for example according to the so-called HGMS (High Gradient Magnetic Separation) technique.

5. Method according to Claim 1, **characterized in that** the said receptor and the said other receptor contain the same ligand, immobilized respectively on the said support and the said other support.

6. Method according to Claim 1, according to which the reagent comprises particles of the support conjugated with the receptor, having the capacity to be separated from any liquid medium in which they are dispersed, under the action of a physical means applied to the said liquid medium, **characterized in that** the said physical means is applied in relationship with the said other support, in order to concentrate the said deposit in the said useful zone.

7. Method according to Claim 1, **characterized in that** the reagent is labelled, and the treatment of the analyte comprises a step of determining the marker for the deposit concentrated, immobilized in the said useful zone of the said other support.

8. Method according to Claim 7, **characterized in that** the labelling of the reagent is obtained according to at least one of the following modes, namely:
- the support itself constitutes a marker
- a marker is bound to the said support
- a marker is bound to the said receptor.

9. Method according to Claim 7, **characterized in that** the analyte is determined from the deposit concentrated in the said useful zone, by any method chosen from the group including topographical methods, for example by atomic force microscopy (abbreviated AFM), magnetic methods, for example by magnetic force microscopy, electrical methods, for example measurement of a variation in capacitance or resistance, optical methods, for example measurement of the refractive index, and methods for measuring mass variations, for example quartz crystal microbalance.

10. Method according to Claim 9, **characterized in that** the useful zone is designed so that the quantity of the deposit immobilized on the said other support is at least equal to the sensitivity threshold of the method of determination, expressed as number of particles per unit of surface.

11. Method according to Claim 1, according to which the assay format is direct, **characterized in that** the capture partner is the said complex between the said support, the receptor and the analyte.

12. Method according to Claim 1, according to which the assay format is by competition, **characterized in that** the capture partner is the said reagent.

13. Method according to Claim 1, **characterized in that**, from the intermediate medium, another sample is obtained, by enriching the said intermediate medium with complex, and the said other sample is brought into contact with the accessible surface of the said other support.

14. Method according to Claim 13, **characterized in that** the said other sample is brought into contact with the accessible surface of the said other support, by depositing a measured quantity of the said other sample on the accessible surface of the latter.

15. Method according to Claim 13, **characterized in that** the said other sample is brought into contact with the said accessible surface of the said other support, by passing a stream of the said other sample, of a relatively small thickness, optionally recycled, in contact with the said accessible surface.

16. Method according to Claim 1, according to which the liquid medium contains a plurality of various analytes, **characterized in that** the accessible surface of the said other support comprises a plurality of useful zones, exhibiting a plurality of other receptors which are respectively different, of capture partners which are respectively different.
